# EUROPEAN PATENT SPECIFICATION

(11) **EP 1 159 659 B1**
(45) Date of publication and mention of the grant of the patent: **21.01.2004**
(21) Application number: 99905655.9
(22) Date of filing: 02.02.1999
(51) Int. Cl.: G05F 1/10, H03K 17/16, H02M 3/07, G05F 3/24, G05F 3/20

(54) **FOUR-PHASE CHARGE PUMP WITH LOWER PEAK CURRENT**
VIERPHASENLADUNGSPUMPE MIT GERINGEM SPITZENSTROM
POMPE DE CHARGE A QUATRE PHASES AVEC FAIBLE COURANT DE CRETE

(43) Date of publication of application: 05.12.2001
(73) Proprietor: Macronix International Co., Ltd., Hsinchu (TW)
(72) Inventor: LIN, Yu Shen, Taipei, Taiwan 104 (TW); HUNG, Chun-Hsiung, Hsinchu, Taiwan 300 (TW); WAN, Ray-Lin, Fremont, CA 94539 (US)
(74) Representative: Horner, David Richard
(86) International application number: PCT/US1999/002259
(87) International publication number: WO 2000/046648

(56) References cited:
- EP-A- 0 594 293
- WO-A-97/23037
- US-A- 5 585 765
- US-A- 5 589 793
- US-A- 5 818 289

## Description

### BACKGROUND OF THE INVENTION

### Field of the Invention

This invention relates to the field of charge pump circuits, and to integrated circuits using charge pumps to produce on-chip voltages outside the range of the off-chip voltage supply.

### Description of the Related Art

A charge pump generates a great deal of power supply and ground noise at times when peak currents drive the charge pump. In the event that the power supply line or the ground line is shared between the charge pump and another analog circuit block on the same chip, the other analog circuit block will suffer from the power supply or ground noise generated by the charge pump. Further, flash memory architecture and mixed mode integrated circuit architecture often has a power supply or ground line shared between a charge pump and another analog circuit block. What is needed is a charge pump that generates less power supply and ground noise, especially when the power supply line and/or the ground line is shared between the charge pump and another analog circuit.

Figure 1 illustrates a block diagram of a charge pump 100. In Figure 1, clock signal circuitry 200 provides pump timing signals 210 to a pump timing circuit 300. Pump timing circuit 300 provides amplified pump timing signals to pump stages 400. Pump timing circuit 300 is coupled to a voltage supply 302 and a ground 304.

Figure 2 schematically illustrates the pump timing circuit 300. The pump timing circuit 300 includes four series of inverters: a first inverter series 310, a second inverter series 330, a third inverter series 350, and a fourth inverter series 370.

The first inverter series 310 includes an input 312, a first inverter 314, a second inverter 316, a third inverter 318, a fourth inverter 320, and an output 322. The input 312 is connected to the input of the first inverter 314. The first inverter 314, the second inverter 316, the third inverter 318, and the fourth inverter 320 are connected in series. The output of the fourth inverter 320 is connected to the output 322. The output 322 provides an amplified first pump clock signal 324.

The second inverter series 330 includes an input 332, a fifth inverter 334, a sixth inverter 336, a seventh inverter 338, an eighth inverter 340, and an output 342. The input 332 is connected to the input of the fifth inverter 334. The fifth inverter 334, the sixth inverter 336, the seventh inverter 338, and the eighth inverter 340 are connected in series. The output of the eighth inverter 340 is connected to the output 342. The output 342 provides an amplified second transfer clock signal 344.

The third inverter series 350 includes an input 352, a ninth inverter 354, a tenth inverter 356, an eleventh inverter 358, a twelfth inverter 360, and an output 362. The input 352 is connected to the input of the ninth inverter 354. The ninth inverter 354, the tenth inverter 356, the eleventh inverter 358, and the twelfth inverter 360 are connected in series. The output of the twelfth inverter 360 is connected to the output 362. The output 362 provides an amplified second pump clock signal 364.

The fourth inverter series 370 includes an input 372, a thirteenth inverter 374, a fourteenth inverter 376, a fifteenth inverter 378, a sixteenth inverter 380, and an output 382. The input 372 is connected to the input ofthe thirteenth inverter 374. The thirteenth inverter 374, the fourteenth inverter 376, the fifteenth inverter 378, and the sixteenth inverter 380 are connected in series. The output of the sixteenth inverter 380 is connected to the output 382. The output 382 provides an amplified first transfer clock signal 384.

The following table details the length and width dimensions of the p-channel and n-channel transistors for some of the inverters in the pump timing circuit 300.

| Inverter | P-channel width (µm) | P-channel length (µm) | N-channel width (µm) | N-channel length (µm) |
|---|---|---|---|---|
| fourth inverter 320 | 800 | 0.5 | 300 | 0.5 |
| twelfth inverter 360 | 800 | 0.5 | 300 | 0.5 |

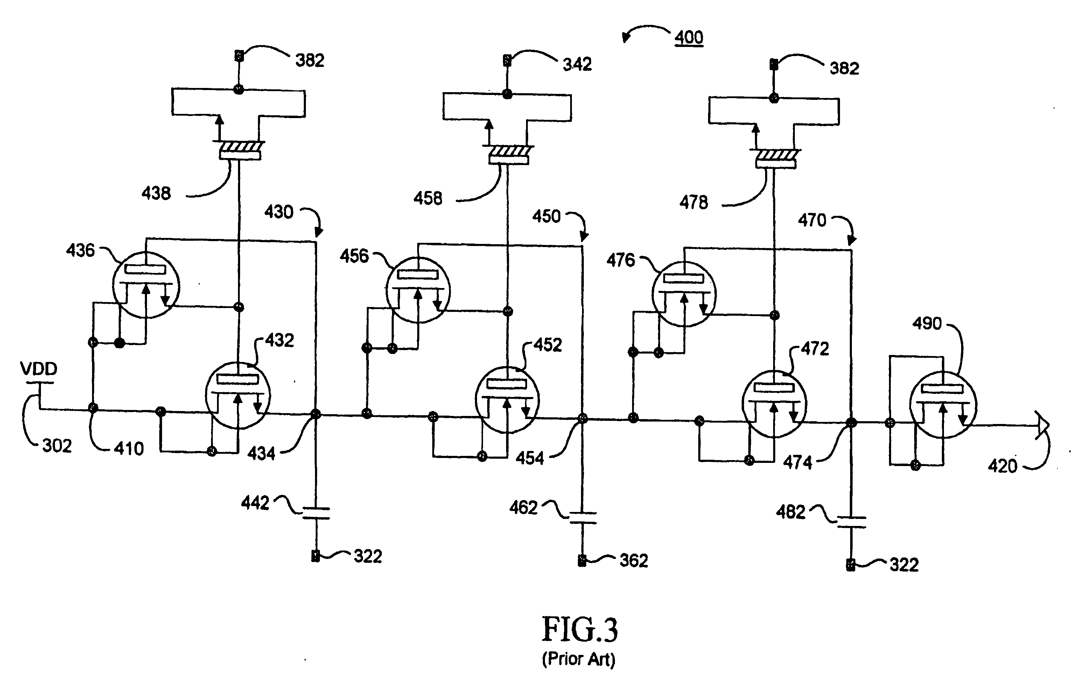

Figure 3 schematically illustrates pump stages 400 shown in US-A-6,100,557. Pump stages 400 includes an input 410, a first stage 430, a second stage 450, a third stage 470, a diode 490, and an output 420. The input 410, the first stage 430, the second stage 450, the third stage 470, the diode 490, and the output 420 are connected in series. The input 410 is coupled to the voltage supply 302 (VDD) and the first stage 430.

The first stage 430 includes a first transistor 432, a second transistor 436, a first transfer capacitor 438, and a first pump capacitor 442. The first transistor 432 is an n-channel transistor having a gate, a source connected to node 434, and a drain connected to the input 410. The second transistor 436 is an n-channel transistor having a gate connected to node 434, a source connected to the gate of the first transistor 432, and a drain connected to the drain of the first transistor 432. The first transfer capacitor 438 is a capacitor-connected n-channel transistor having a first terminal connected to the fourth inverter series output 382 and a second terminal connected to the gate of the first transistor 432. The first pump capacitor 442 has a first terminal connected to the first inverter series output 322 and a second terminal connected to node 434.

The second stage 450 includes a third transistor 452, a fourth transistor 456, a second transfer capacitor 458, and a second pump capacitor 462. The third transistor 452 is an n-channel transistor having a gate, a source connected to node 454, and a drain connected to node 434. The fourth transistor 456 is an n-channel transistor having a gate connected to node 454, a source connected to the gate of the third transistor 452, and a drain connected to the drain of the third transistor 452. The second transfer capacitor 458 is a capacitor-connected n-channel transistor having a first terminal connected to the second inverter series output 342 and a second terminal connected to the gate of the third transistor 452. The second pump capacitor 462 has a first terminal connected to the third inverter series output 362 and a second terminal connected to node 454.

The third stage 470 includes a fifth transistor 472, a sixth transistor 476, a third transfer capacitor 478, and a third pump capacitor 482. The fifth transistor 472 is an n-channel transistor having a gate, a source connected to node 474, and a drain connected to node 454. The sixth transistor 476 is an n-channel transistor having a gate connected to node 474, a source connected to the gate of the fifth transistor 472, and a drain connected to the drain of the fifth transistor 472. The third transfer capacitor 478 is a capacitor-connected n-channel transistor having a first terminal connected to the fourth inverter series output 382 and a second terminal connected to the gate of the fifth transistor 472. The third pump capacitor 482 has a first terminal connected to the first inverter series output 322 and a second terminal connected to node 474.

Diode 490 is a diode-connected n-channel transistor having a first terminal connected to node 474 and a second terminal connected to the pump stages output 420.

US-A-5,589,793 discloses a charge pump circuit with improved frequency stability. The charge pump has cascaded stages and a control circuit for generating four timing signals.

EP 0 594 293 A2 discloses a charge pump circuit with improved voltage regulation of the voltage output by the charge pump. The charge pump is driven by four timing signals.

WO 97/23037 discloses a charge pump circuit having improved power efficiency. The charge pump is driven by two clock signals.

Heretofore, the requirement for a charge pump with less power supply noise and less ground noise has not been fully met. What is needed is a solution that simultaneously addresses both of these requirements.

### SUMMARY OF THE INVENTION

Aspects of the present invention are set out in claims 1, 26 and 31.

A primary goal of the invention is to provide a charge pump that has less power supply noise. Another primary goal of the invention is to provide a charge pump having less ground noise. Another primary goal of the invention is to provide a charge pump which overcomes inefficiencies of older designs.

A charge pump of an embodiment of the present invention comprises a first timing circuit supplying a timing signal from a timing signal output and each of the charge pump stages receive a timing signal via a capacitor, charge is pumped to an output in response to a timing signal made of two components separated by a delay. Current usually flows in the same direction during both components of the timing signal, and the power of the timing signal increases from the onset of the second component. The power of the timing signal increases due to the use of the second component to couple the capacitor to a voltage source, a current source, or a ground.

Acoording to another embodiment of the invention, the charge pump pumps charge in response to several timing signals. In a preferred embodiment, the onsets of the first and second components are defined by distinct edges of the timing signals. The onsets can be defined by several clock signals.

In another embodiment of the invention, two transistors and two capacitors form a charge pump boost stage. In a preferred embodiment, two clock signals drive one of the capacitors. An amplification circuit can increase the power of one or both of the clock signals, and a diode can be coupled to the output of the charge pump.

In further embodiments of the invention, a second charge pump stage is added and a third charge pump stage is added to define a four phase charge pump. In yet further embodiments ofthe invention, one or both of two transfer clock signals can be used to drive one or more of the pump capacitors in the four-phase charge pump. According to yet other embodiments of the invention, an integrated circuit is provided including the charge pump on a single chip.

A method for reducing a magnitude of a peak current flowing in a charge pump according to one embodiment comprises driving a charge pump node with a first timing signal having a polarity, and driving the node with a second timing signal having the polarity, such that the onsets of the first and second timing signals are separated by a delay. Another method for reducing a magnitude of a peak current flowing in a charge pump according to one embodiment comprises driving a charge pump node by activating a current handling device coupled the node through a capacitor, and after a delay, driving the node by activating a second current handling device coupled to the node through the capacitor, while the first current handling device remains activated.

### BRIEF DESCRIPTION OF THE FIGURES

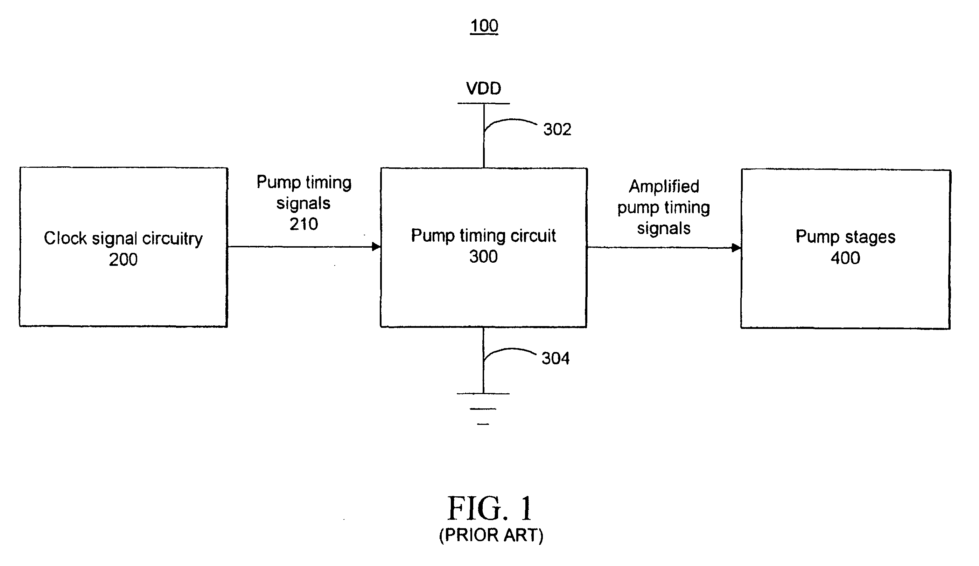

Figure 1 is a block diagram of a known charge pump.

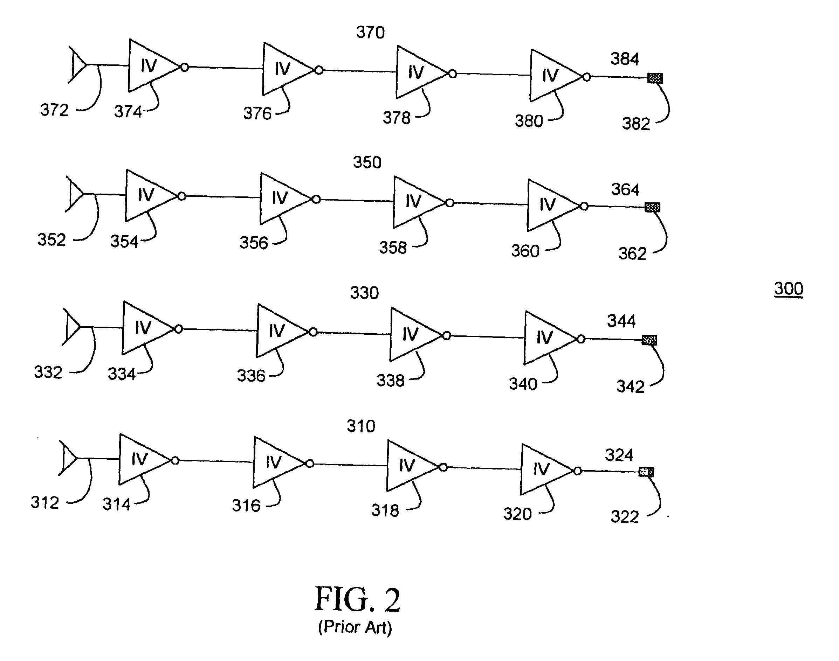

Figure 2 is a circuit diagram of a known pump timing circuit.
Figure 3 is a circuit diagram of a known series of pump stages.

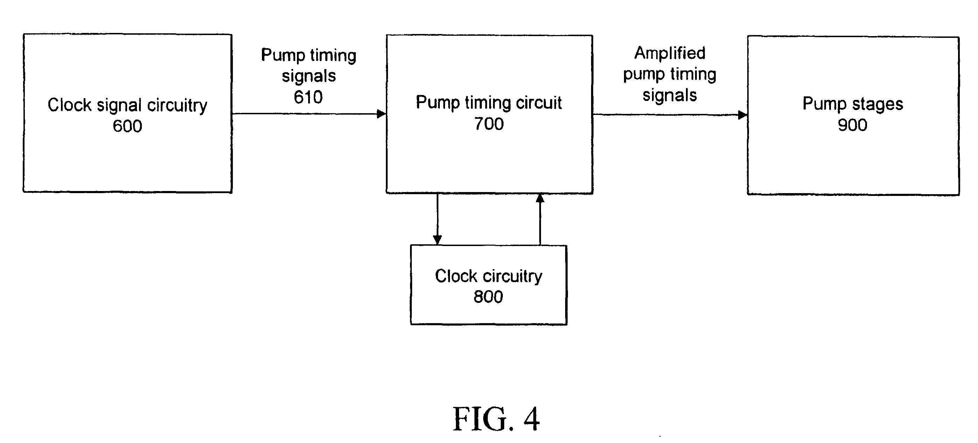

Figure 4 is a block diagram of a charge pump representing an embodiment of the invention.

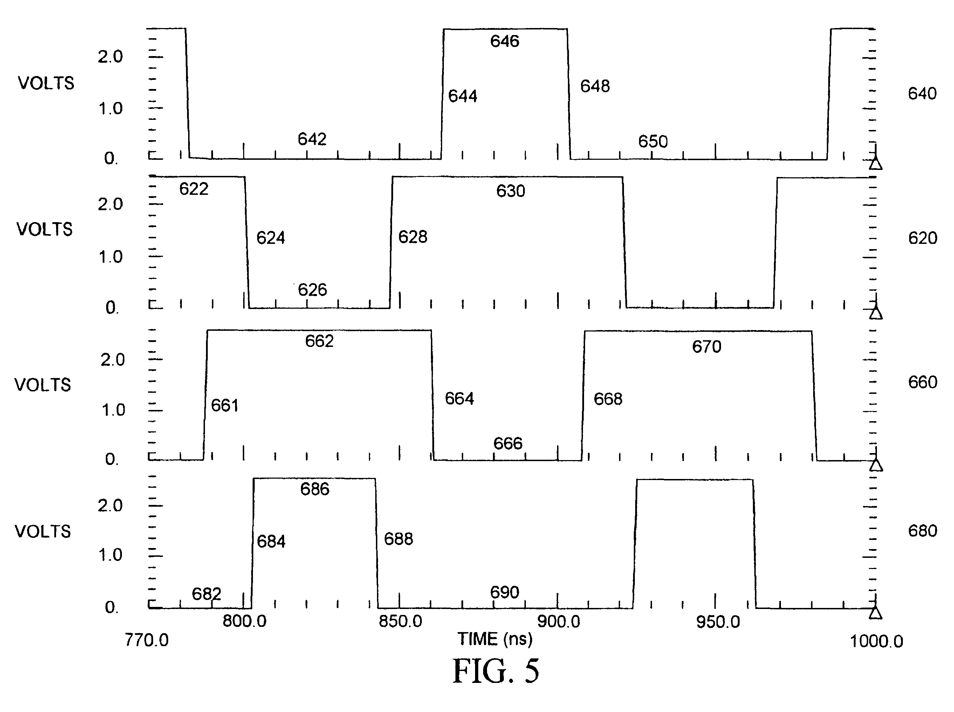

Figure 5 is a timing diagram illustrating pump timing signals.

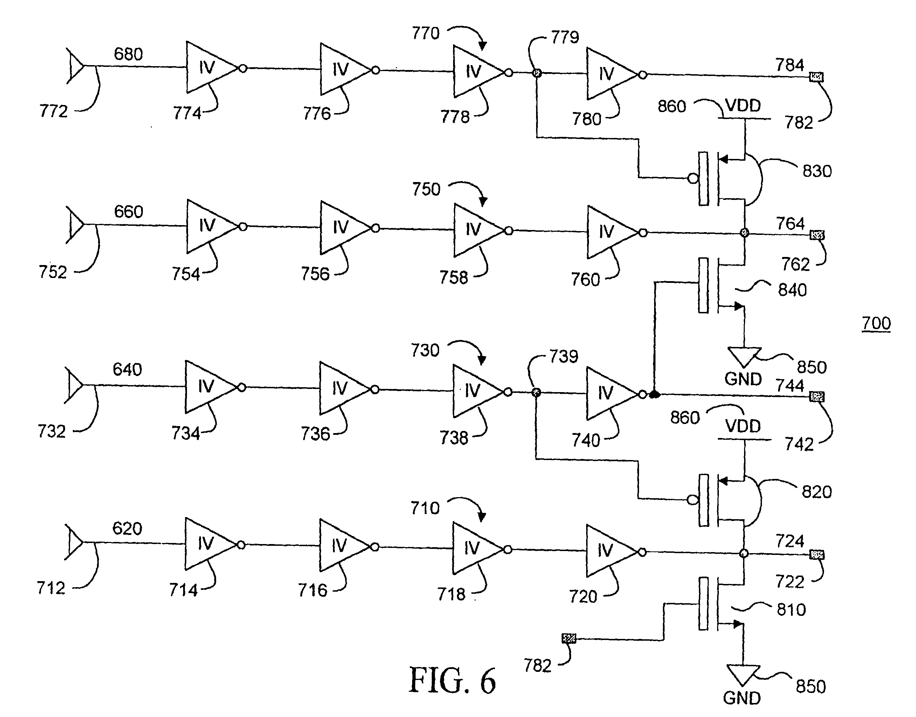

Figure 6 is a circuit diagram of a pump timing circuit and clock circuitry.

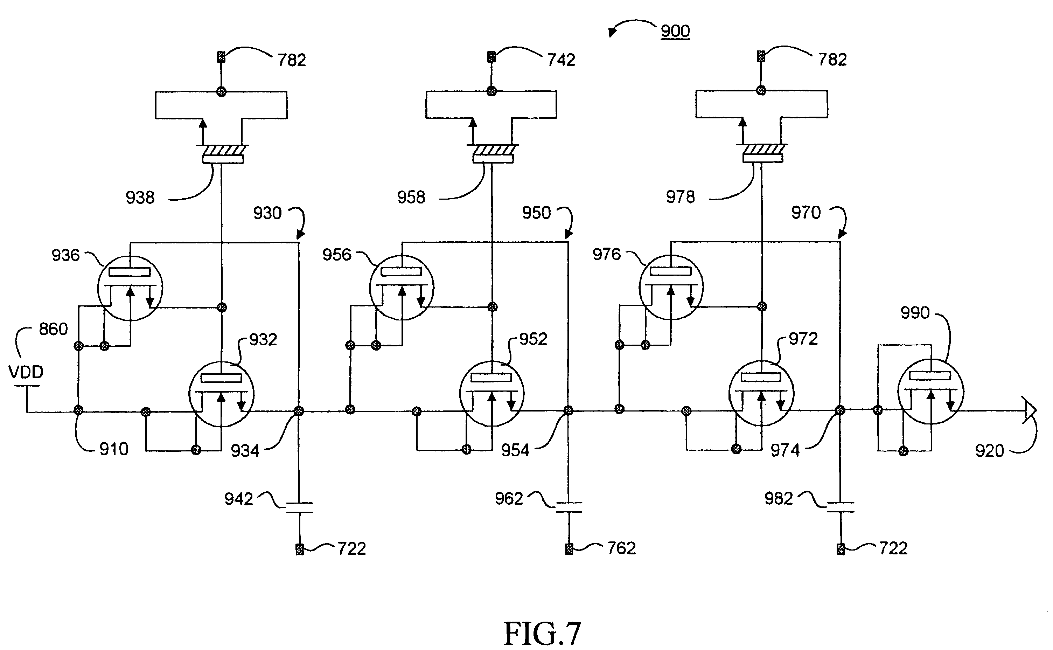

Figure 7 is a circuit diagram of pump stages.

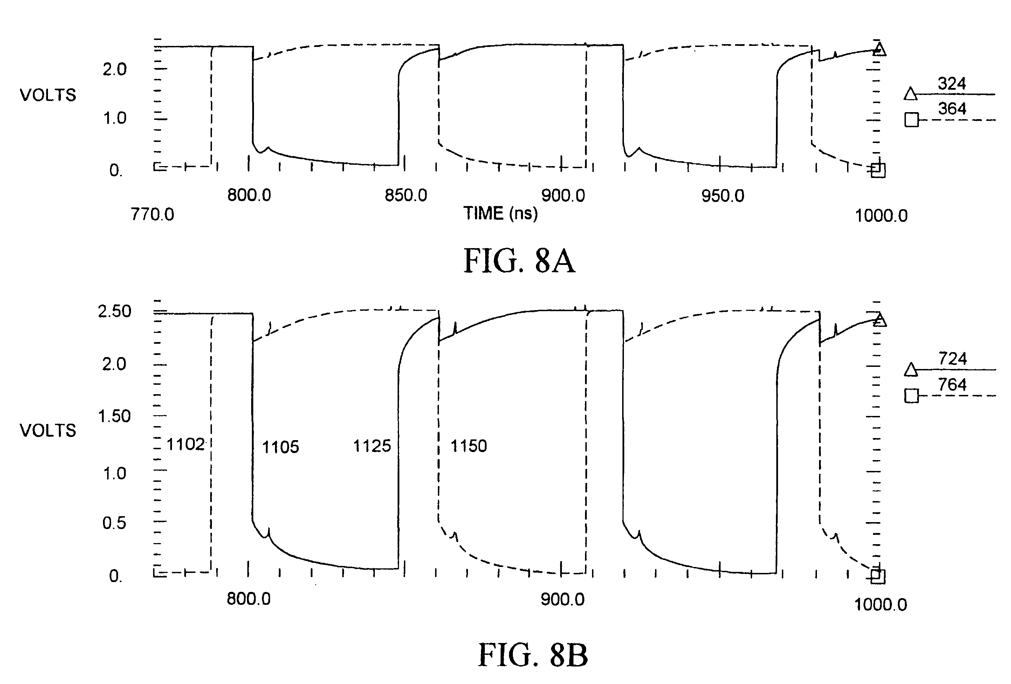

Figure 8A is a timing diagram of amplified pump timing signals supplied by a pump timing circuit.
Figure 8B is a timing diagram of amplified pump timing signals supplied by a pump timing circuit.

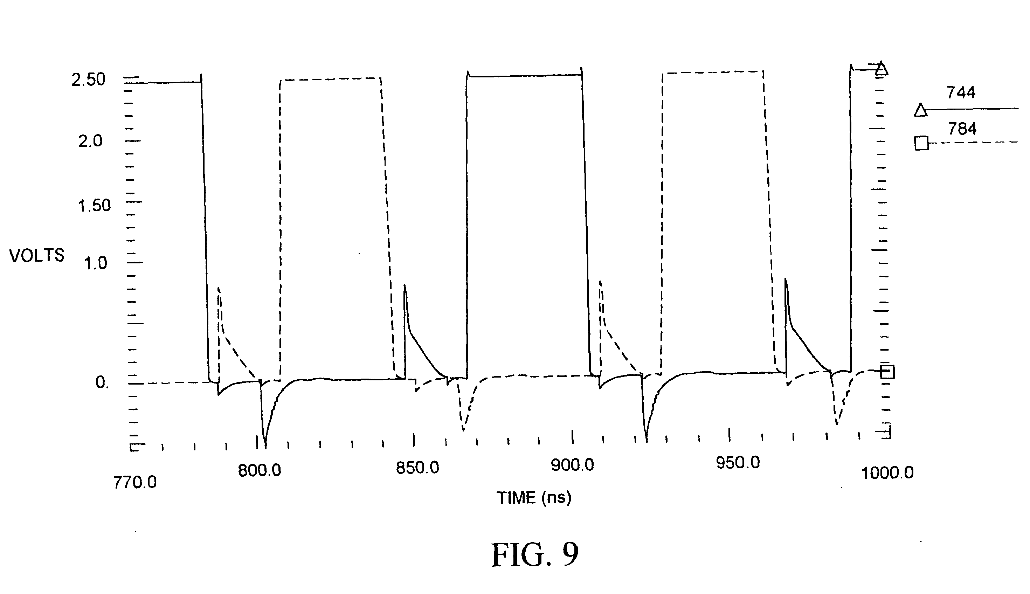

Figure 9 is a timing diagram of amplified pump timing signals supplied by a pump timing circuit.

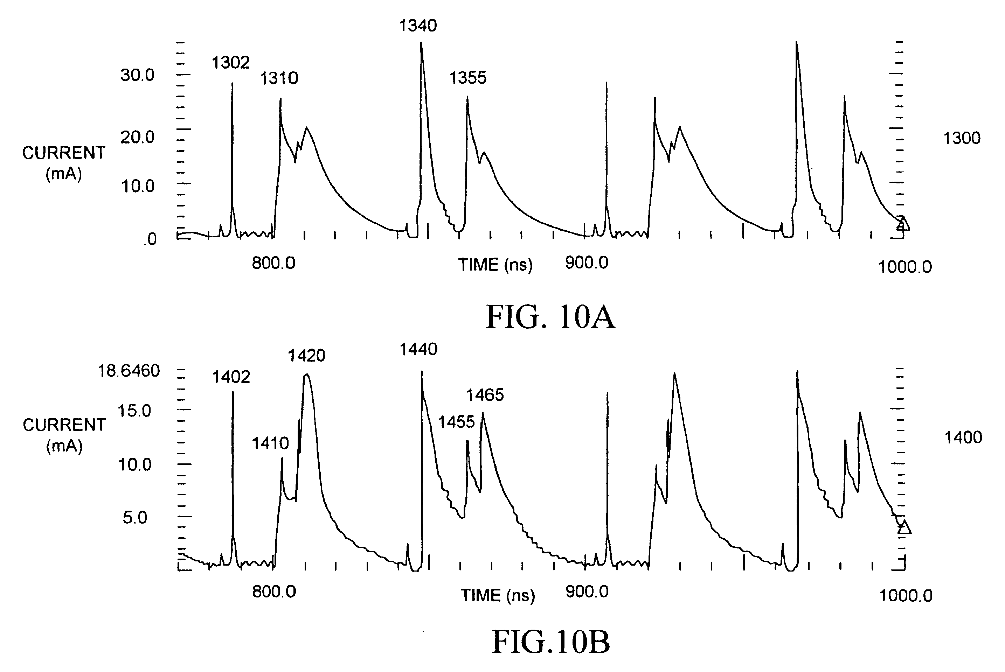

Figure 10A is a timing diagram of current supplied by the voltage supply of a known charge pump.
Figure 10B is a timing diagram of current supplied by the voltage supply of a charge pump.
Figure 11A is a timing diagram of current sunk by the ground of a known charge pump.

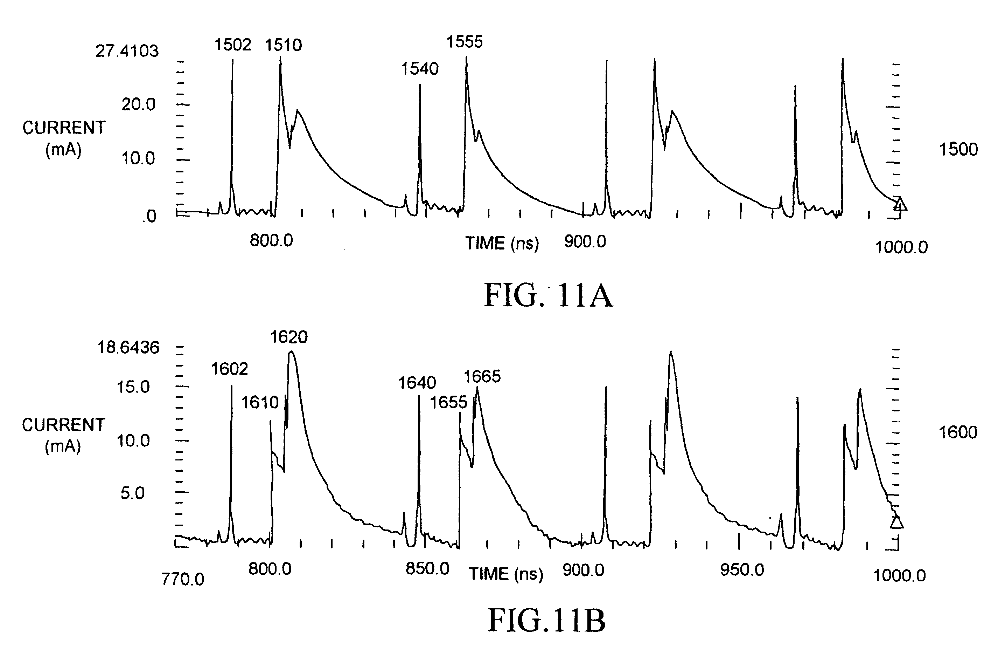

Figure 11B is a timing diagram of current sunk by the ground of a charge pump.

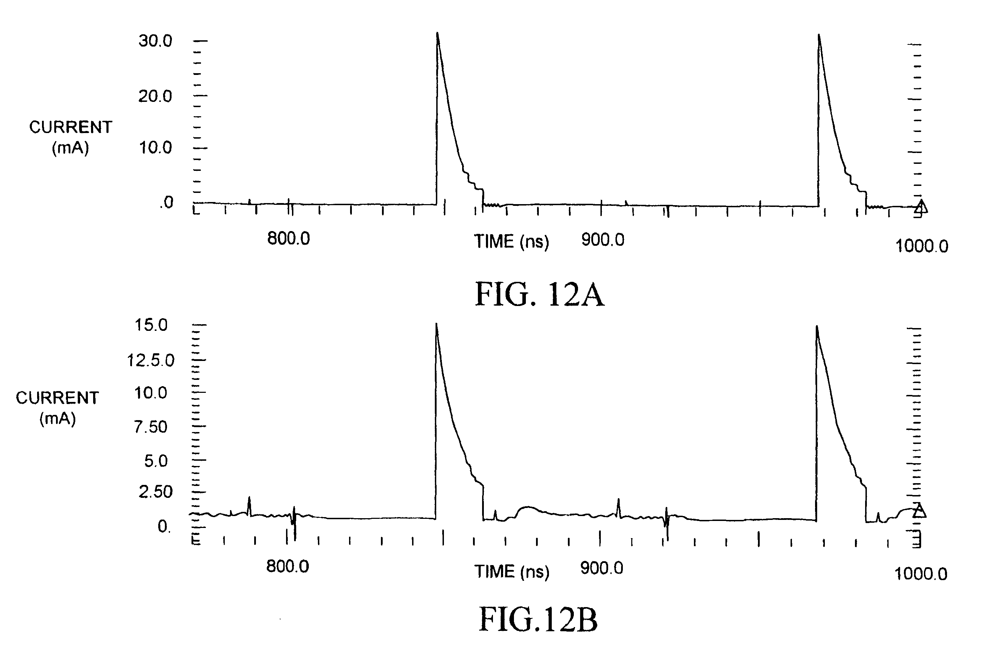

Figure 12A is a timing diagram of current supplied by the output of a known charge pump.
Figure 12B is a timing diagram of current supplied by the output of a charge pump.

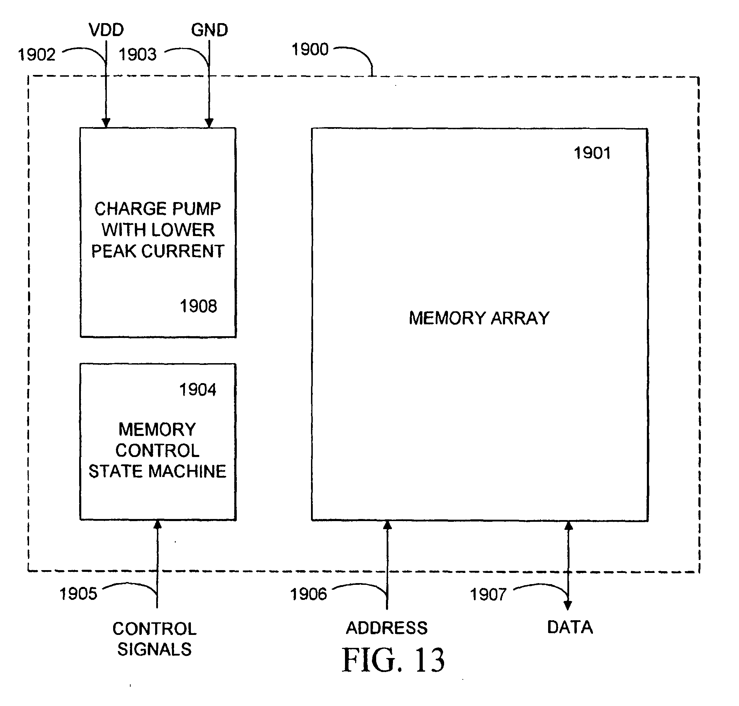

Figure 13 is a simplified block diagram of an integrated circuit utilizing a four-phase charge pump with lower peak current.

All figures showing timing diagrams illustrate the same time frame, from about 770 nanoseconds to about 1 microsecond.

### DETAILED DESCRIPTION

Figure 4 illustrates a block diagram of the charge pump with lower peak current according to the present invention. In Figure 4, a clock signal circuitry 600 provides pump timing signals 610 to a pump timing circuit 700. Pump timing circuit 700 provides amplified pump timing signals to pump stages 900. Clock circuitry 800 processes signals internal to the pump timing circuit 700 and contributes to processing the pump timing signals 610 into amplified pump timing signals.

Figure 5 is a voltage versus time timing diagram of pump timing signals 610 provided by clock signal circuitry 600. Pump timing signals 610 include four periodic pulse trains: a first pump clock signal 620, a second transfer clock signal 640, a second pump clock signal 660, and a first transfer clock signal 680. Parts of the first pump clock signal 620 are high level 622, falling edge 624, low level 626, rising edge 628, and high level 630. Parts of the second transfer clock signal 640 are low level 642, rising edge 644, high level 646, falling edge 648, and low level 650. Parts of the second pump clock signal 660 are rising edge 661, high level 662, falling edge 664, low level 666, rising edge 668, and high level 670. Parts of the first transfer clock signal 680 are low level 682, rising edge 684, high level 686, falling edge 688, and low level 690.

Figure 6 schematically illustrates pump timing circuit 700 and clock circuitry 800. Pump timing circuit 700 produces amplification by stepping through inverters of generally increasing transistor widths. Pump timing circuit 700 includes four series of inverters: a first inverter series 710, a second inverter series 730, a third inverter series 750, and a fourth inverter series 770.

The first inverter series 710 includes an input 712, a first inverter 714, a second inverter 716, a third inverter 718, a fourth inverter 720, and an output 722. The input 712 receives the first pump clock signal 620. The input 712 is connected to the input of the first inverter 714. The first inverter 714, the second inverter 716, the third inverter 718, and the fourth inverter 720 are connected in series. The output of the fourth inverter 720 is connected to the output 722. The output 722 provides an amplified first pump clock signal 724.

The second inverter series 730 includes an input 732, a fifth inverter 734, a sixth inverter 736, a seventh inverter 738, an eighth inverter 740, and an output 742. The input 732 receives the second transfer clock signal 640. The input 732 is connected to the input of the fifth inverter 734. The fifth inverter 734, the sixth inverter 736, the seventh inverter 738, and the eighth inverter 740 are connected in series. The output of the seventh inverter 738 and the input of the eighth inverter 740 are connected to node 739. The output of the eighth inverter 740 is connected to the output 742. The output 742 provides an amplified second transfer clock signal 744.

The third inverter series 750 includes an input 752, a ninth inverter 754, a tenth inverter 756, an eleventh inverter 758, a twelfth inverter 760, and an output 762. The input 752 receives the second pump clock signal 660. The input 752 is connected to the input of the ninth inverter 754. The ninth inverter 754, the tenth inverter 756, the eleventh inverter 758, and the twelfth inverter 760 are connected in series. The output of the twelfth inverter 760 is connected to the output 762. The output 762 provides an amplified second pump clock signal 764.

The fourth inverter series 770 includes an input 772, a thirteenth inverter 774, a fourteenth inverter 776, a fifteenth inverter 778, a sixteenth inverter 780, and an output 782. The input 772 receives the first transfer clock signal 680. The input 772 is connected to the input of the thirteenth inverter 774. The thirteenth inverter 774, the fourteenth inverter 776, the fifteenth inverter 778, and the sixteenth inverter 780 are connected in series. The output of the fifteenth inverter 778 and the input of the sixteenth inverter 780 are connected to node 779. The output of the sixteenth inverter 780 is connected to the output 782. The output 782 provides an amplified first transfer clock signal 784.

The following table details examples of the length and width dimensions of the p-channel and n-channel transistors for the inverters in the pump timing circuit 700. Of course, the invention is not limited to these examples.

| Inverter | P-channel width (µm) | P-channel length (µm) | N-channel width (µm) | N-channel length (µm) |
|---|---|---|---|---|
| first inverter 714 | 6 | 0.5 | 3 | 0.5 |
| second inverter 716 | 26 | 0.5 | 13 | 0.5 |
| third inverter 718 | 80 | 0.5 | 40 | 0.5 |
| fourth inverter 720 | 400 | 0.5 | 100 | 0.5 |
| fifth inverter 734 | 6 | 0.5 | 3 | 0.5 |
| sixth inverter 736 | 26 | 0.5 | 13 | 0.5 |
| seventh inverter 738 | 80 | 0.5 | 40 | 0.5 |
| eighth inverter 740 | 40 | 0.5 | 20 | 0.5 |
| ninth inverter 754 | 6 | 0.5 | 3 | 0.5 |
| tenth inverter 756 | 26 | 0.5 | 13 | 0.5 |
| eleventh inverter 758 | 80 | 0.5 | 40 | 0.5 |
| twelfth inverter 760 | 400 | 0.5 | 100 | 0.5 |
| thirteenth inverter 774 | 6 | 0.5 | 3 | 0.5 |
| fourteenth inverter 776 | 26 | 0.5 | 13 | 0.5 |
| fifteenth inverter 778 | 80 | 0.5 | 40 | 0.5 |
| sixteenth inverter 780 | 40 | 0.5 | 20 | 0.5 |

The second inverter series 730 and the fourth inverter series 770 are coupled to the first inverter series output 722 and the third inverter series output 762 through routing transistors. Clock circuitry 800 includes a first routing transistor 810, a second routing transistor 820, a third routing transistor 830, a fourth routing transistor 840, a ground 850, and a voltage supply 860. The first routing transistor 810 is an n-channel transistor with a gate connected to the fourth inverter series output 782, a source connected to ground 850, and a drain connected to the first inverter series output 722. The second routing transistor 820 is a p-channel transistor with a gate connected to node 739, a source connected to voltage supply 860, and a drain connected to the first inverter series output 722. The third routing transistor 830 is a p-channel transistor with a gate connected to node 779, a source connected to voltage supply 860, and a drain connected to the third inverter series output 762. The fourth routing transistor 840 is an n-channel transistor with a gate connected to the second inverter series output 742, a source connected to ground 850, and a drain connected to the third inverter series output 762.

The following table details examples of the length and width dimensions of the p-channel and n-channel transistors for the routing transistors in clock circuitry 800. Of course, the invention is not limited to these examples.

| Transistor | Width (µm) | Length (µm) |
|---|---|---|
| first routing transistor 810 | 200 | 0.5 |
| second routing transistor 820 | 400 | 0.5 |
| third routing transistor 830 | 400 | 0.5 |
| fourth routing transistor 840 | 200 | 0.5 |

It can be appreciated that in the above examples, the sum of the widths of the first routing transistor 810 and the n-channel transistor in the fourth inverter 720 corresponds to the width of the n-channel transistor in the fourth inverter 320. The sum of the width of the second routing transistor 820 and the p-channel transistor in the fourth inverter 720 corresponds to the width of the p-channel transistor in the fourth inverter 320. The sum of the widths of the third routing transistor 830 and the p-channel transistor in the twelfth inverter 760 corresponds to the width of the p-channel transistor in the twelfth inverter 360. The sum of the widths of the fourth routing transistor 840 and the n-channel transistor in the twelfth inverter 760 corresponds to the width of the n-channel transistor in the twelfth inverter 360.

Figure 7 schematically illustrates pump stages 900. Triple well transistors are indicated with a circled transistor. Transistors having a thick gate oxide are indicated by a transistor with a rectangle for the gate. Transistors having a lower threshold voltage due to masking during implantation of extra impurities are indicated by a transistor with a hatched rectangle.

Pump stages 900 includes an input 910, a first stage 930, a second stage 950, a third stage 970, a diode 990, and an output 920. The input 910, the first stage 930, the second stage 950, the third stage 970, the diode 990, and the output 920 are connected in series. The input 910 is coupled to the voltage supply 860 and the first stage 930.

The first stage 930 includes a first transistor 932, a second transistor 936, a first transfer capacitor 938, and a first pump capacitor 942. The first transistor 932 is an n-channel triple well transistor with a thick gate oxide having a gate, a source connected to node 934, and a drain connected to the input 910. The second transistor 936 is an n-channel triple well transistor with a thick gate oxide having a gate connected to node 934, a source connected to the gate of the first transistor 932, and a drain connected to the drain of the first transistor 932. The first transfer capacitor 938 is a capacitor-connected n-channel transistor with a thick gate oxide and a lower threshold voltage having a first terminal connected to the fourth inverter series output 782 and a second terminal connected to the gate of the first transistor 932. The first pump capacitor 942 is a 200 picofarad capacitor having a first terminal connected to the first inverter series output 722 and a second terminal connected to node 934.

The second stage 950 includes a third transistor 952, a fourth transistor 956, a second transfer capacitor 958, and a second pump capacitor 962. The third transistor 952 is an n-channel triple well transistor with a thick gate oxide having a gate, a source connected to node 954, and a drain connected to node 934. The fourth transistor 956 is an n-channel triple well transistor with a thick gate oxide having a gate connected to node 954, a source connected to the gate of the third transistor 952, and a drain connected to the drain of the third transistor 952. The second transfer capacitor 958 is a capacitor-connected n-channel transistor with a thick gate oxide and a lower threshold voltage having a first terminal connected to the second inverter series output 742 and a second terminal connected to the gate of the third transistor 952. The second pump capacitor 962 is a 200 picofarad capacitor having a first terminal connected to the third inverter series output 762 and a second terminal connected to node 954.

The third stage 970 includes a fifth transistor 972, a sixth transistor 976, a third transfer capacitor 978, and a third pump capacitor 982. The fifth transistor 972 is an n-channel triple well transistor with a thick gate oxide having a gate, a source connected to node 974, and a drain connected to node 954. The sixth transistor 976 is an n-channel triple well transistor with a thick gate oxide having a gate connected to node 974, a source connected to the gate of the fifth transistor 972, and a drain connected to the drain of the fifth transistor 972. The third transfer capacitor 978 is a capacitor-connected n-channel transistor with a thick gate oxide and a lower threshold voltage having a first terminal connected to the fourth inverter series output 782 and a second terminal connected to the gate of the fifth transistor 972. The third pump capacitor 982 is a 200 picofarad capacitor having a first terminal connected to the first inverter series output 722 and a second terminal connected to node 974.

Diode 990 is a diode-connected n-channel triple well transistor with a thick gate oxide having a first terminal connected to node 974 and a second terminal connected to the pump stages output 920.

The following table details some examples of the length and width dimensions of the n-channel transistors in pump stages 900. Of course, the invention is not limited to the examples.

| Transistor | Width (µm) | Length (µm) |
|---|---|---|
| first transistor 932 | 130 | 0.8 |
| second transistor 936 | 20 | 0.8 |
| first transfer capacitor 938 | 40 | 40 |
| third transistor 952 | 130 | 0.8 |
| fourth transistor 956 | 20 | 0.8 |
| second transfer capacitor 958 | 40 | 40 |
| fifth transistor 972 | 130 | 0.8 |
| sixth transistor 976 | 20 | 0.8 |
| third transfer capacitor 978 | 40 | 40 |
| diode 990 | 130 | 0.8 |

Figure 8A is a timing diagram displaying voltage versus time for the amplified first pump clock signal 324 and the amplified second pump clock signal 364 provided by pump timing circuit 300. Figure 8B is a timing diagram displaying voltage versus time for the amplified first pump clock signal 724 and the amplified second pump clock signal 764 provided by pump timing circuit 700.

Figure 9 is a timing diagram displaying voltage versus time for the amplified second transfer clock signal 744 and the amplified first transfer clock signal 784 provided by pump timing circuit 700.

Figure 10A is a timing diagram displaying current versus time for current signal 1300 supplied by voltage supply 302. Figure 10B is a timing diagram displaying current versus time for current signal 1400 supplied by voltage supply 860.

Figure 11A is a timing diagram displaying current versus time for current sunk by ground 304. Figure 11B is a timing diagram displaying current versus time for current signal 1600 sunk by ground 850.

Referring to Figures 4-11B, falling edge 624 of the first pump clock signal 620 is received by first inverter series input 712 and processed by the first inverter series 710, resulting in falling edge 1105 of the amplified first pump clock signal 724 driven by the fourth inverter 720. Falling edge 1105 of the amplified first pump clock signal 724 is capacitively coupled to node 934 through the first pump capacitor 942 and to node 974 through third pump capacitor 982. Then, the voltages of node 934 and node 974 drop. Low level 626 of first pump clock signal 620 is similarly processed, and the voltages of node 934 and node 974 continue to be driven down. Thus, the occurrence of falling edge 624 causes current spike 1410 in current signal 1400 and current spike 1610 in current signal 1600.

Following a time delay after falling edge 624 of the first pump clock signal 620, rising edge 684 of first transfer clock signal 680 is received by the fourth inverter series input 772. The rising edge 684 is processed by the fourth inverter series 770 until the fourth inverter series output 782, and further processed by the first routing transistor 810. The first routing transistor 810 helps the fourth inverter 720 to drive down the voltages of node 934 and node 974. High level 686 of first transfer clock signal 680 is similarly processed, and the voltages of node 934 and node 974 continue to be driven down. Thus, rising edge 684 of first transfer clock signal 680 combined with low level 626 of the first pump clock signal 620 result in current spike 1420 in current signal 1400 and current spike 1620 in current signal 1600.

Driving down the voltages of node 934 and node 974 in two components separated by a time delay in the above manner yields advantageous results. Specifically, the current signal spikes are significantly lower. Driving down the voltages of node 934 and node 974 in two components yields current signal 1400 having current spike 1410 and current spike 1420 with respective magnitudes of about 11 mA and 18.6 mA. In contrast, driving down the voltages of node 934 and node 974 in one component yields current signal 1300 having a peak 1310 with a much higher magnitude of about 25 mA. Similarly, driving down the voltages of node 934 and node 974 in two components yields current signal 1600 having current spike 1610 and current spike 1620 with respective magnitudes of about 12 mA and 18.6 mA. In contrast, driving down the voltages of node 934 and node 974 in one component yields current signal 1500 having a peak 1510 with a much higher magnitude of about 27.4 mA.

Rising edge 628 of the first pump clock signal 620 is received by first inverter series input 712 and processed by the first inverter series 710, resulting in rising edge 1125 of the amplified first pump clock signal 724 driven by the fourth inverter 720. Rising edge 1125 of the amplified first pump clock signal 724 is capacitively coupled to node 934 through the first pump capacitor 942 and to node 974 through third pump capacitor 982. Then, the voltages of node 934 and node 974 rise. High level 630 of first pump clock signal 620 is similarly processed, and the voltages of node 934 and node 974 continue to be driven up. Thus, the occurrence of rising edge 628 causes current spike 1440 in current signal 1400 and current spike 1640 in current signal 1600.

Following a time delay after rising edge 628 of the first pump clock signal 620, rising edge 644 of second transfer clock signal 640 is received by the second inverter series input 732. The rising edge 644 is processed by the second inverter series 730 until node 739, and further processed by the second routing transistor 820. The second routing transistor 820 helps the fourth inverter 720 to drive up the voltages of node 934 and node 974. High level 646 of second transfer clock signal 640 is similarly processed, and the voltages of node 934 and node 974 continues to be driven up. Thus, rising edge 644 of second transfer clock signal 640 combined with high level 630 of the first pump clock signal 620 result in current spike 1465 in current signal 1400 and current spike 1665 in current signal 1600.

Driving up the voltages of node 934 and node 974 in two components separated by a time delay in the above manner yields advantageous results. Specifically, the current spikes are much lower. Driving up the voltages of node 934 and node 974 in two components yields current signal 1400 having current spike 1440 and current spike 1465 with respective magnitudes of about 18.6 mA and 15 mA. In contrast, driving up the voltages of node 934 and node 974 in one component yields current signal 1300 having a peak 1340 with a much higher magnitude of about 36 mA. Similarly, driving up the voltages of node 934 and node 974 in two components yields current signal 1600 having current spike 1640 and current spike 1665 with respective magnitudes of about 13 mA and 15 mA. In contrast, driving up the voltages of node 934 and node 974 in one component yields current signal 1500 having a peak 1540 with a much higher magnitude of about 22 mA.

Falling edge 664 of the second pump clock signal 660 is received by third inverter series input 752 and processed by the third inverter series 750, resulting in falling edge 1150 of the amplified second pump clock signal 764 driven by the twelfth inverter 760. Falling edge 1150 of the amplified second pump clock signal 764 is capacitively coupled to node 954 through the second pump capacitor 962. Then, the voltage of node 954 drops. Low level 666 of the second pump clock signal 660 is similarly processed, and the voltage of node 954 continues to be driven down. Thus, the occurrence of falling edge 664 causes current spike 1455 in current signal 1400 and current spike 1655 in current signal 1600.

Following a time delay after falling edge 664 of the second pump clock signal 660, rising edge 644 of second transfer clock signal 640 is received by the second inverter series input 732. The rising edge 644 is processed by the second inverter series 730 until the second inverter series output 742, and further processed by the fourth routing transistor 840. The fourth routing transistor 840 helps the twelfth inverter 760 to drive down the voltage of node 954. High level 646 of second transfer clock signal 640 is similarly processed, and the voltage of node 954 continues to be driven down. Thus, rising edge 644 of second transfer clock signal 640 combined with low level 666 of the second pump clock signal 660 results in current spike 1465 in current signal 1400 and current spike 1665 in current signal 1600.

Driving down the voltage of node 954 in two components separated by a time delay in the above manner yields advantageous results. Specifically, the current signal spikes are a lot lower. Driving down the voltage of node 954 in two components yields current signal 1400 having current spike 1455 and current spike 1465 with respective magnitudes of about 13 mA and 15 mA. In contrast, driving down the voltage of node 954 in one component yields current signal 1300 having a peak 1355 with a much higher magnitude of about 26 mA. Similarly, driving down the voltage of node 954 in two components yields current signal 1600 having current spike 1655 and current spike 1665 with respective magnitudes of about 11 mA and 15 mA. In contrast, driving down the voltage of node 954 in one component yields current signal 1500 having a peak 1555 with a much higher magnitude of about 27 mA.

Rising edge 661 of the second pump clock signal 660 is received by third inverter series input 752 and processed by the third inverter series 750, resulting in rising edge 1102 of the amplified second pump clock signal 764 driven by the twelfth inverter 760. Rising edge 1102 of the amplified second pump clock signal 764 is capacitively coupled to node 954 through the second pump capacitor 962. Then, the voltage of node 954 rises. High level 662 of the second pump clock signal 660 is similarly processed, and the voltage of node 954 continues to be driven up. Thus, the occurrence of rising edge 661 causes current spike 1402 in current signal 1400 and current spike 1602 in current signal 1600.

Following a time delay after rising edge 661 of the second pump clock signal 660, rising edge 684 of the first transfer clock signal 680 is received by the fourth inverter series input 772. The rising edge 684 is processed by the fourth inverter series 770 until node 779, and further processed by the third routing transistor 830. The third routing transistor 830 helps the twelfth inverter 760 to drive up the voltage of node 954. High level 686 of first transfer clock signal 680 is similarly processed, and the voltage of node 954 continues to be driven up. Thus, rising edge 684 of the first transfer clock signal 680 combined with high level 662 of the second pump clock signal 660 results in current spike 1420 in current signal 1400 and current spike 1620 in current signal 1600.

Driving up the voltage of node 954 in two components separated by a time delay in the above manner yields advantageous results. Again, the current signal spikes are much lower. Driving up the voltage of node 954 in two components yields current signal 1400 having current spike 1402 and current spike 1420 with respective magnitudes of about 16 mA and 18.6 mA. In contrast, driving up the voltage of node 954 in one component yields current signal 1300 having a peak 1302 with a much higher magnitude of about 26 mA. Similarly, driving up the voltage of node 954 in two components yields current signal 1600 having current spike 1602 and current spike 1620 with respective magnitudes of about 15 mA and 18.6 mA. In contrast, driving up the voltage of node 954 in one component yields current signal 1500 having a peak 1502 with a much higher magnitude of about 26 mA.

Figure 12A is a timing diagram displaying current versus time for current supplied by pump stages output 420. Figure 12B is a timing diagram displaying current versus time for current supplied by pump stages output 920.

Figure 13 provides a simplified diagram of an integrated circuit utilizing the charge pump with lower peak current of the present invention. The integrated circuit 1900 includes a semiconductor substrate. A memory array 1901 is included on the device which utilizes operating voltages which are outside the pre-specified range of the supply potential normally applied to the device at supply terminals 1902 and 1903, which are adapted to receive a supply potential VDD and ground.

The integrated circuit in this example includes a memory control state machine 1904, which establishes various operational modes for the memory array 1901. Input signals include control signals 1905 applied to the control state machine 1904, address signals 1906 applied to the memory array circuitry, and data signals 1907 also applied to the memory array 1901. According to the present invention, there is a charge pump with lower peak current 1908 included on the device which is adapted to receive the supply potentials VDD and ground.

Figure 13 is representative of a wide variety of integrated circuits which include on-chip circuitry that utilizes operational voltages outside the pre-specified range of the supply potential. Memory devices such as flash memory devices are one class of integrated circuits according to the present invention.

Other embodiments of the invention can use other transistor sizes, for example, a different ratio between the widths of the routing transistors and the widths of the transistors in the inverters, and different oxide thicknesses. Another embodiment of the invention is a negative charge pump. Another embodiment of the invention drives charge pump nodes in two components triggered by a single signal. The single signal triggers the first component, and a delayed part of the single signal triggers the second component.

The foregoing description of various embodiments of the invention has been presented for purposes of illustration and description. It is not intended to limit the invention to the precise forms disclosed. Many modifications and equivalent arrangements will be apparent.

## Claims

1. A charge pump, comprising:
a pump timing circuit (700) having a timing signal output, and the timing signal output, supplying a timing signal; and
a plurality of serially arranged charge pump stages (930, 950, 970) including respective transfer circuits and pump nodes (934, 954, 974), the pump node of one said plurality of charge pump stages coupled to the timing signal output via a capacitor (942, 962, 982), said plurality of serially arranged charge pump stages having a charge pump output (920), wherein charge is pumped to the charge pump output through the plurality of charge pump stages in response to the timing signal, and the timing signal is driven by a first timing signal component and a second timing signal component, and an onset of said first timing signal component is separated by a delay from an onset of said second timing signal component.

2. The charge pump of claim 1, wherein a direction of current flow of the timing signal remains constant during both the first timing signal component and the second timing signal component.

3. The charge pump of claim 1, wherein a power of the timing signal increases due to the onset of the second timing signal component.

4. The charge pump of claim 3, wherein the power of the timing signal increases due to the onset of the second timing signal component by connecting the capacitor to at least one of a voltage source, a current source, and a ground.

5. The charge pump of claim 1, wherein the charge pumped to the charge pump output is positive.

6. The charge pump as claimed in Claim 1, wherein
said pump timing circuit comprises a pump timing circuit having a plurality of timing signal inputs (712, 732, 752, 772) and a plurality of timing signal outputs (722, 742, 762, 782) the plurality of timing signal inputs receiving a plurality of pump timing signals (620, 640, 660, 680) and a plurality of timing signal outputs supplying a plurality of amplified pump timing signals (724, 744, 764, 784); and
said plurality of serially arranged charge pump stages comprises a plurality of serially arranged charge pump stages including respective transfer circuits and pump nodes, the pump nodes of said plurality of charge pump stages respectively coupled to the plurality of timing signal outputs via a plurality of capacitors, said plurality of serially arranged charge pump stages having a charge pump output, wherein charge is pumped to the charge pump output through the plurality of charge pump stages in response to the plurality of amplified pump timing signals, and at least one of the plurality of amplified pump timing signals is driven by a first timing signal component and a second timing signal component, and an onset of said first timing signal component is separated by a delay from an onset of said second timing signal component.

7. The charge pump of claim 6, wherein the onset of the first timing signal component and the onset of the second timing signal component are defined by distinct transitional edges defined by two of the plurality of pump timing signals.

8. The charge pump of claim 6, wherein the onset of the first timing signal component and the onset of the second timing signal component are defined by a plurality of clock signals.

9. The charge pump of claim 6, wherein a direction of current flow of the at least one of the plurality of amplified pump timing signals remains constant during both the first timing signal component and the second timing signal component.

10. The charge pump of claim 6, wherein a power of the at least one of the plurality of amplified pump timing signals increases due to the onset of the second timing signal component.

11. The charge pump of claim 10, wherein the power of the at least one of the plurality of amplified pump timing signals increases by connecting one of the capacitors to at least one of a voltage source, a current source, and a ground.

12. The charge pump as claimed in claim 1, wherein a first of said plurality of serially arranged charge pump stages (930) comprises:
a first pump capacitor (942) having a first terminal and a second terminal, and the first terminal of the first pump capacitor coupled to a first pump clock signal (724);
a first transfer capacitor (938) having a first terminal and a second terminal, and the first terminal of the first transfer capacitor coupled to a first transfer clock signal (784);
a first transistor (932) adapted to transfer charge to the second terminal of the first pump capacitor, and the first transistor having a gate, a first terminal, and a second terminal, and the gate of the first transistor connected to the second terminal of the first transfer capacitor, and the second terminal of the first transistor connected to the second terminal of the first pump capacitor;
a second transistor (936) adapted to selectively couple the gate of the first transistor to the first terminal of the first transistor, and the second transistor having a gate, a first terminal, and a second terminal, and the gate of the second transistor connected to the second terminal of the first pump capacitor, and the second terminal of the second transistor connected to the gate of the first transistor, and the first terminal of the second transistor connected to the first terminal of the first transistor; and
wherein said pump timing circuit comprises:
clock circuitry coupled to a) the first transfer clock signal and b) the first terminal of the first pump capacitor.

13. The charge pump of claim 12, wherein the clock circuitry includes a routing transistor (810) having a gate, a first terminal, and a second terminal, and the gate of the routing transistor coupled to the first transfer clock signal, and the first terminal of the routing transistor coupled to the first terminal of the first pump capacitor, and the second terminal of the routing transistor coupled to one of a voltage source, a current source, and a ground.

14. The charge pump of claim 12, further comprising an amplification circuit coupled to at least one of the first pump clock signal and the first transfer clock signal, wherein the amplification circuit increases the power of the at least one of the first pump clock signal and the first transfer clock signal.

15. The charge pump of claim 12, further comprising a diode having a first terminal and a second terminal, and the first terminal of the diode coupled to the second terminal of the first pump capacitor.

16. A charge pump as claimed in claim 1, wherein a first of said plurality of serially arranged charge pump stages (930) comprises:
a first pump capacitor (942) having a first terminal and a second terminal, and the first terminal of the first pump capacitor coupled to a first pump clock signal (724);
a first transfer capacitor (938) having a first terminal and a second terminal, and the first terminal of the first transfer capacitor coupled to a first transfer clock signal (754);
a first transistor (932) adapted to transfer charge to the second terminal of the first pump capacitor, and the first transistor having a gate, a first terminal, and a second terminal, and the gate of the first transistor connected to the second terminal of the first transfer capacitor, and the second terminal of the first transistor connected to the second terminal of the first pump capacitor;
a second transistor (936) adapted to selectively couple the gate of the first transistor to the first terminal of the first transistor, and the second transistor having a gate, a first terminal, and a second terminal, and the gate of the second transistor connected to the second terminal of the first pump capacitor, and the second terminal of the second transistor connected to the gate of the first transistor, and the first terminal of the second transistor connected to the first terminal of the first transistor, and
wherein a second of said plurality of serially arranged charge pump stages (950) comprises: a second pump capacitor (962) having a first terminal and a second terminal, and the first terminal of the second pump capacitor coupled to a second pump clock signal (764);
a second transfer capacitor (758) having a first terminal and a second terminal, and the first terminal of the second transfer capacitor coupled to a second transfer clock signal (744);
a third transistor (952) adapted to transfer charge to the second terminal of the second pump capacitor, and the third transistor having a gate, a first terminal, and a second terminal, and the gate of the third transistor connected to the second terminal of the second transfer capacitor, and the first terminal of the third transistor connected to the second terminal of the first transistor, and the second terminal of the third transistor connected to the second terminal of the second pump capacitor;
a fourth transistor (956) adapted to selectively couple the gate of the third transistor to the first terminal of the third transistor, and the fourth transistor having a gate, a first terminal, and a second terminal, and the gate of the fourth transistor connected to the second terminal of the second pump capacitor, and the second terminal of the fourth transistor connected to the gate of the third transistor, and the first terminal of the fourth transistor connected to the first terminal of the third transistor; and
wherein said pump timing circuit comprises clock circuitry including one or more of:
a) a first routing transistor (810) having a gate, a first terminal, and a second terminal, and the gate of the first routing transistor coupled to the first transfer clock signal, and the first terminal of the first routing transistor coupled to the first terminal of the first pump capacitor, and the second terminal of the first routing transistor coupled to one of a voltage source, a current source, and a ground;
b) a second routing transistor (820) having a gate, a first terminal, and a second terminal, and the gate of the second routing transistor coupled to the second second transfer clock signal, and the first terminal of the second routing transistor coupled to the first terminal of the first pump capacitor, and the second terminal of the second routing transistor coupled to one of a voltage source, a current source, and a ground;
c) a third routing transistor (830) having a gate, a first terminal, and a second terminal, and the gate of the third routing transistor coupled to the first transfer clock signal, and the first terminal of the third routing transistor coupled to the first terminal of the second pump capacitor, and the second terminal of the third routing transistor coupled to one of a voltage source, a current source, and a ground; and
d) a fourth routing transistor (840) having a gate, a first terminal, and a second terminal, and the gate of the fourth routing transistor coupled to the second transfer clock signal, and the first terminal of the fourth routing transistor coupled to the first terminal of the second pump capacitor, and the second terminal of the fourth routing transistor coupled to one of a voltage source, a current source, and a ground.

17. The charge pump of claim 16, further comprising an amplification circuit coupled to at least one of the first pump clock signal, the first transfer clock signal, the second pump clock signal, and the second transfer clock signal, wherein the amplification circuit increases the power of the at least one of the first pump clock signal, the first transfer clock signal, the second pump clock signal, and the second transfer clock signal.

18. The charge pump of claim 16, further comprising at least one of: a) an amplification circuit (770) coupling the first transfer clock signal to the gate of the third routing transistor, b) an amplification circuit (730) coupling the second transfer clock signal to the gate of the second routing transistor, c) an amplification circuit (710) coupling the first transfer clock signal to the gate of the first routing transistor, and d) an amplification circuit (750) coupling the second transfer clock signal to the gate of the fourth routing transistor.

19. The charge pump of claim 17, wherein the amplification circuit includes one or more of
a) a first plurality of inverters (910) including:
a first inverter (714) having an input and an output, and the input of the first inverter coupled to the first pump clock signal;
a second inverter (716) having an input and an output, and the input of the second inverter connected to the output of the first inverter,
a third inverter (718) having an input and an output, and the input of the third inverter connected to the output of the second inverter;
a fourth inverter (720) having an input and an output, and the input of the fourth inverter connected to the output of the third inverter, and the output of the fourth inverter connected to the first terminal of the first pump capacitor;
b) a second plurality of inverters (730) including:
a fifth inverter (734) having an input and an output, and the input of the fifth inverter coupled to the second transfer clock signal;
a sixth inverter (736) having an input and an output, and the input of the sixth inverter connected to the output of the fifth inverter;
a seventh inverter (738) having an input and an output, and the input of the seventh inverter connected to the output of the sixth inverter,
an eigth inverter (740) having an input and an output, and the input of the eighth inverter connected to the output of the seventh inverter, and the output of the eighth inverter connected to the first terminal of the second transfer capacitor,
c) a third plurality of inverters (750) including:
a ninth inverter (754) having an input and an output, and the input of the ninth inverter coupled to the second pump clock signal; a tenth inverter (756) having an input and an output, and the input of the tenth
inverter connected to the output of the ninth inverter; an eleventh inverter (758) having an input and an output, and the input of the
eleventh inverter connected to the output of the tenth inverter; a twelfth inverter (760) having an input and an output, and the input of the
twelfth inverter connected to the output of the eleventh inverter, and the output of the twelfth inverter connected to the first terminal of the second pump capacitor; and
d) a fourth plurality of inverters (770) including:
a thirteenth inverter (774) having an input and an output, and the input of the thirteenth inverter coupled to the first transfer clock signal;
a fourteenth inverter (776) having an input and an output, and the input of the fourteenth inverter connected to the output of the thirteenth inverter;
a fifteenth inverter (778) having an input and an output, and the input of the fifteenth inverter connected to the output of the fourteenth inverter;
a sixteenth inverter (780) having an input and an output, and the output of the sixteenth inverter connected to the output of the fifteenth inverter, and the output of the sixteenth inverter connected to the first terminal of the first transfer capacitor.

20. The charge pump of claim 19, wherein the gate of the first routing transistor is connected to the output of the sixteenth inverter, the gate of the second routing transistor is connected to the output of the seventh inverter, the gate of the third routing transistor is connected to the output of the fifteenth inverter, and the gate of the fourth routing transistor is connected to the output of the eighth inverter.

21. The charge pump as claimed in claim 1, wherein a first of said plurality of serially arranged charge pump stages (930) comprises:
a first pump capacitor (942) having a first terminal and a second terminal, and the first terminal of the first pump capacitor coupled to a first pump clock signal (724);
a first transfer capacitor (938) having a first terminal and a second terminal, and the first terminal of the first transfer capacitor coupled to a first transfer clock signal (784);
a first transistor (932) adapted to transfer charge to the second terminal of the first pump capacitor, and the first transistor having a gate, a first terminal, and a second terminal, and the gate of the first transistor connected to the second terminal of the first transfer capacitor, and the second terminal of the first transistor connected to the second terminal of the first pump capacitor;
a second transistor (936) adapted to selectively couple the gate of the first transistor to the first terminal of the first transistor, and the second transistor having a gate, a first terminal, and a second terminal, and the gate of the second transistor connected to the second terminal of the first pump capacitor, and the second terminal of the second transistor connected to the gate of the first transistor, and the first terminal of the second transistor connected to the first terminal of the first transistor; and
wherein a second of said plurality of serially arranged charge pump stages (950) comprises:
a second pump capacitor (962) having a first terminal and a second terminal, and the first terminal of the second pump capacitor coupled to a second pump clock signal;
a second transfer capacitor (958) having a first terminal and a second terminal, and the first terminal of the second transfer capacitor coupled to a second transfer clock signal;
a third transistor (952) adapted to transfer charge to the second terminal of the second pump capacitor, and the third transistor having a gate, a first ' terminal, and a second terminal, and the gate of the third transistor connected to the second terminal of the second transfer capacitor, and the first terminal of the third transistor connected to the second terminal of the first transistor, and the second terminal of the third transistor connected to the second terminal of the second pump capacitor,
a fourth transistor (956) adapted to selectively couple the gate of the third transistor to the first terminal of the third transistor, and the fourth transistor having a gate, a first terminal, and a second terminal, and the gate of the fourth transistor connected to the second terminal of the second pump capacitor, and the second terminal of the fourth transistor connected to the gate of the third transistor, and the first terminal of the fourth transistor connected to the first terminal of the third transistor; and
wherein a third of said plurality of serially arranged charge pump stages (970) comprises:
a third pump capacitor (982) having a first terminal and a second terminal, and the first terminal of the third pump capacitor coupled to the first pump clock signal;
a third transfer capacitor (978) having a first terminal and a second terminal, and the first terminal of the third transfer capacitor coupled to the first transfer clock signal;
a fifth transistor (972) adapted to transfer charge to the second terminal of the third pump capacitor, and the fifth transistor having a gate, a first terminal, and a second terminal, and the gate of the fifth transistor connected to the second terminal of the third transfer capacitor, and the first terminal of the fifth transistor connected to the second terminal of the third transistor, and the second terminal of the fifth transistor connected to the second terminal of the third pump capacitor;
a sixth transistor (976) adapted to selectively couple the gate of the fifth transistor to the first terminal of the fifth transistor, and the sixth transistor having a gate, a first terminal, and a second terminal, and the gate of the sixth transistor connected to the second terminal of the third pump capacitor, and the second terminal of the sixth transistor connected to the gate of the fifth transistor, and the first terminal of the sixth transistor connected to the first terminal of the fifth transistor; and
wherein said timing circuit comprises clock circuitry including one or more of:
a) a first routing transistor (810) having a gate, a first terminal, and a second terminal, and the gate of the first routing transistor coupled to the first transfer clock signal, and the first terminal of the first routing transistor coupled to at least one of i) the first terminal of the first pump capacitor and ii) the first terminal of the third pump capacitor, and the second terminal of the first routing transistor coupled to one of a voltage source, a current source, and a ground;
b) a second routing transistor (820) having a gate, a first terminal, and a second terminal, and the gate of the second routing transistor coupled to the second transfer clock signal, and the first terminal of the second routing transistor coupled to at least one of i) the first terminal of the first pump capacitor and ii) the first terminal of the third pump capacitor, and the second terminal of the second routing transistor coupled to one of a voltage source, a current source, and a ground;
c) a third routing transistor (830) having a gate, a first terminal, and a second terminal, and the gate of the third routing transistor coupled to the first transfer clock signal, and the first terminal of the third routing transistor coupled to the first terminal of the second pump capacitor, and the second terminal of the third routing transistor coupled to one of a voltage source, a current source, and a ground; and
d) a fourth routing transistor (840) having a gate, a first terminal, and a second terminal, and the gate of the fourth routing transistor coupled to the second transfer clock signal, and the first terminal of the fourth routing transistor coupled to the first terminal of the second pump capacitor, and the second terminal of the fourth routing transistor coupled to one of a voltage source, a current source, and a ground.

22. The multiple stage charge pump of claim 21, further comprising an amplification circuit coupled to at least one of the first pump clock signal, the first transfer clock signal, the second pump clock signal, and the second transfer clock signal, wherein the amplification circuit increases the power of the at least one of the first pump clock signal, the first transfer clock signal, the second pump clock signal, and the second transfer clock signal.

23. The multiple stage charge pump of claim 21, further comprising at least one of: a) an amplification circuit (770) coupling the first transfer clock signal to the gate of the third routing transistor, b) an amplification circuit (730) coupling the second transfer clock signal to the gate of the second routing transistor, c) an amplification circuit (710) coupling the first transfer clock signal to the gate of the first routing transistor, and d) an amplification circuit (750) coupling the second transfer clock signal to the gate of the fourth routing transistor.

24. The multiple stage charge pump of claim 22, wherein the amplification circuit includes one or more of:
a) a first plurality of inverters (910) including:
a first inverter (714) having an input and an output, and the input of the first inverter coupled to the first pump clock signal;
a second inverter (716) having an input and an output, and the input of the second inverter connected to the output of the first inverter;
a third inverter (718) having an input and an output, and the input of the third inverter connected to the output of the second inverter,
a fourth inverter (720) having an input and an output, and the input of the fourth inverter connected to the output of the third inverter, and the output of the fourth inverter connected to at least one of i) the first terminal of the first pump capacitor and ii) the first terminal of the third pump capacitor,
b) a second plurality of inverters (730) including:
a fifth inverter (734) having an input and an output, and the input of the fifth inverter coupled to the second transfer clock signal;
a sixth inverter (736) having an input and an output, and the input of the sixth inverter connected to the output of the fifth inverter;
a seventh inverter (738) having an input and an output, and the input of the seventh inverter connected to the output of the sixth inverter;
an eighth inverter (740) having an input and an output, and the input of the eighth inverter connected to the output of the seventh inverter, and the output of the eighth inverter connected to the first terminal of the second transfer capacitor;
c) a third plurality of inverters (750) including:
a ninth inverter (754) having an input and an output, and the input of the ninth inverter coupled to the second pump clock signal;
a tenth inverter (756) having an input and an output, and the input of the tenth inverter connected to the output of the ninth inverter;
an eleventh inverter (758) having an input and an output, and the input of the eleventh inverter connected to the output of the tenth inverter,
a twelfth inverter (760) having an input and an output, and the input of the twelfth inverter connected to the output of the eleventh inverter, and the output of the twelfth inverter connected to the first terminal of the second pump capacitor, and
d) a fourth plurality of inverters (770) including:
a thirteenth inverter (774) having an input and an output, and the input of the thirteenth inverter coupled to the first transfer clock signal;
a fourteenth inverter (776) having an input and an output, and the input of the fourteenth inverter connected to the output of the thirteenth inverter;
a fifteenth inventor (778) having an input and an output, and the input of the fifteenth inverter connected to the output of the fourteenth inverter;
a sixteenth inventor (780) having an input and an output, and the input of the sixteenth inverter connected to the output of the fifteenth inverter, and the output of the sixteenth inverter connected to at least one of i) the first terminal of the first transfer capacitor and ii) the first terminal of the third transfer capacitor.

25. The multiple stage charge pump of claim 24, wherein the gate of the first routing transistor is connected to the output of the sixteenth inverter, the gate of the second routing transistor is connected to the output of the seventh inverter, the gate of the third routing transistor is connected to the output of the fifteenth inverter, and the gate of the fourth routing transistor is connected to the output of the eighth inverter.

26. An integrated circuit, comprising:
a semiconductor substrate; a pump timing circuit (700) on the substrate having a timing signal output, and the timing signal output supplying a timing signal; and
a plurality of serially arranged charge pump stages (930,950,970) on the substrate, including respective transfer circuits and pump nodes (934,954,974), and the pump node of one of the plurality of serially connected charge pump stages coupled to the timing signal output via a capacitor (742;962;982), said plurality of serially arranged charge pump stages having a charge pump output (920), wherein charge is pumped to the charge pump output through the plurality of charge pump stages in response to the timing signal, and the timing signal is driven by a first timing signal component and a second timing signal component, and an onset of said first timing signal component is separated by a delay from an onset of said second timing signal component.

27. The integrated circuit of claim 26, wherein a direction of current flow of the timing signal remains constant during both the first timing signal component and the second timing signal component.

28. The integrated circuit of claim 26, wherein a power of the timing signal increases due to the onset of the second timing signal component.

29. The integrated circuit of claim 28, wherein the power of the timing signal increases due to the onset of the second timing signal component by connecting the capacitor to at least one of a voltage source, a current source, and a ground.

30. The integrated circuit as claimed in claim 26, comprising:
a memory array on the substrate; and
a plurality of word lines coupled to the memory array.

31. A method for reducing a magnitude of a peak current flowing in a charge pump, the charge pump having a plurality of serially arranged charge pump stages (930, 950, 970) including respective transfer circuits and
charge pump nodes (934, 954, 974), the method comprising the steps of:
(a) driving a charge pump node in one of said plurality of stages in the charge pump with a first timing signal component; and
(b) driving the charge pump node with a second timing signal component, wherein an onset of the first timing signal component and an onset of the second timing signal component are separated by a delay.

32. The method of claim 31, wherein the particular charge pump node is driven with a greater current capacity following the onset of the second timing signal component

33. The method of claim 31, wherein
step (a) comprises driving a particular charge pump node in one of said plurality of stages by activating a first current handling device coupled to the particular charge pump node through a capacitor, and after a delay; and
step (b) comprises driving the particular charge pump node by activating a second current handling device coupled to the particular charge pump node through the capacitor, while the first current handling device remains activated.

34. The method of claim 33, wherein the first current handling device is a first field effect transistor and the second current handling device is a second field effect transistor.

35. The method of claim 31, wherein the first timing signal component has a polarity and the second timing signal component has the polarity.

## Patentansprüche

1. Ladungspumpe mit:
einem Pumptaktschaltkreis (700), der einen Taktsignalausgang hat, wobei der Taktsignalausgang ein Taktsignal liefert, und
einer Mehrzahl von in Serie angeordneten Ladungspumpenstufen (930, 950, 970), die entsprechende Übertragungsschaltkreise und Pumpenknoten (934, 954, 974) aufweisen, wobei der Pumpenknoten von einer aus der Mehrzahl von Ladungspumpenstufen mit dem Zeittaktsignalausgang über einen Kondensator (942, 962, 982) verbunden ist, die Mehrzahl von seriell angeordneten Ladungspumpenstufen einen Ladungspumpenausgang (920) hat, wobei Ladung in Reaktion auf das Zeittaktsignal durch die Mehrzahl von Ladungspumpenstufen zu dem Ladungspumpenausgang gepumpt wird, und wobei das Zeittaktsignal durch eine erste Zeittaktsignalkomponente und eine zweite Zeittaktsignalkomponente getrieben wird, und beim Einsetzen der ersten Zeittaktsignalkomponente um eine Verzögerung gegenüber dem Einsatz der zweiten Signaltaktkomponente getrennt wird.

2. Ladungspumpe nach Anspruch 1, wobei eine Richtung des Stromflusses des Zeittaktsignales sowohl während der ersten Zeittaktsignalkomponente als auch während der zweiten Zeittaktsignalkomponente konstant bleibt.

3. Ladungspumpe nach Anspruch 1, wobei eine Energie des Zeittaktsignales aufgrund des Einsetzens der zweiten Zeittaktsignalkomponente zunimmt.

4. Ladungspumpe nach Anspruch 3, wobei die Energie des Zeittaktsignales aufgrund des Einsetzens der zweiten Zeittaktsignalkomponente zunimmt, indem der Kondensator eine Verbindung zumindest einem der Folgenden, nämlich einer Spannungsquelle, einer Stromquelle und einer Masse herstellt.

5. Ladungspumpe nach Anspruch 1, wobei die zu dem Ladungspumpenausgang gepumpte Ladung positiv ist.

6. Ladungspumpe nach Anspruch 1, wobei der Pumpenzeittaktschaltkreis einen Pumpenzeittaktschaltkreis aufweist, der eine Mehrzahl von Zeittaktsignaleingängen (712, 732, 752, 772) und eine Mehrzahl von Zeittaktsignalausgängen (722, 742, 762, 782) hat, wobei die Mehrzahl von Zeittaktsignaleingängen eine Mehrzahl von Pumptaktsignalen (620, 640, 660, 680) empfängt und eine Mehrzahl von Zeittaktsignalausgängen eine Mehrzahl verstärkter Taktsignale (724, 744, 764, 784) ausgibt, und
die Mehrzahl von in Serie angeordneten Ladungspumpenstufen eine Mehrzahl von seriell angeordneten Ladungspumpenstufen aufweist, welche entsprechende Übertragungsschattkreise und Pumpenknoten aufweisen, wobei die Pumpenknoten der Mehrzahl von Ladungspumpenstufen jeweils mit der Mehrzahl von Zeittaktsignalausgängen über eine Mehrzahl von Kondensatoren verbunden sind, die Mehrzahl von in Serie angeordneten Ladungspumpenstufen einen Ladungspumpenausgang hat, wobei die Ladung durch die Mehrzahl von Ladungspumpenstufen in Reaktion auf die Mehrzahl verstärkter Pumptaktsignale zu dem Ladungspumpenausgang gepumpt wird, und zumindest eines auf der Mehrzahl von verstärkten Pumptaktsignalen durch eine erste Zeittaktsignalkomponente und eine zweite Zeittaktsignalkomponente getrieben wird, und beim Einsetzen der ersten Zeittaktsignalkomponente um eine Verzögerung gegenüber einem Einsetzen der zweiten Zeittaktsignalkomponente getrennt wird.

7. Ladungspumpe nach Anspruch 6, wobei das Einsetzen der ersten Zeittaktsignalkomponente und das Einsetzen der zweiten Zeittaktsignalkomponente durch getrennte Übergangsflanken definiert werden, die durch zwei aus der Mehrzahl von Pumptaktsignalen definiert werden.

8. Ladungspumpe nach Anspruch 6, wobei das Einsetzen der ersten Zeittaktsignalkomponente und das Einsetzen der zweiten Zeittaktsignalkomponente durch eine Mehrzahl von Taktsignalen definiert werden.

9. Ladungspumpe nach Anspruch 6, wobei eine Richtung des Stromflusses der zumindest einen aus der Mehrzahl verstärkter Taktsignale sowohl während der ersten Zeittaktsignalkomponente als auch während der zweiten Zeittaktsignalkomponente konstant bleibt.

10. Ladungspumpe nach Anspruch 6, wobei die Energie von zumindest einem aus der Mehrzahl von verstärkten Pumptaktsignalen aufgrund des Einsetzens der zweiten Zeittaktsignalkomponente ansteigt.

11. Ladungspumpe nach Anspruch 10, wobei die Energie des zumindest einen aus der Mehrzahl verstärkter Pumptaktsignale durch Verbinden eines der Kondensatoren mit zumindest einem der Folgenden, nämlich einer Spannungsquelle, einer Stromquelle und einer Masse, ansteigt.

12. Ladungspumpe nach Anspruch 1, wobei eine erste der Mehrzahl von in Reihe angeordneten Ladungspumpenstufen (930) aufweist:
einen ersten Pumpenkondensator (942), der einen ersten Anschluß und einen zweiten Anschluß hat, und wobei der erste Anschluß des ersten Pumpenkondensators mit einem ersten Pumptaktsignal (724) verbunden ist,
einen ersten Übertragungskondensator (938), der einen ersten Anschluß und einen zweiten Anschluß hat, wobei der erste Anschluß des ersten Übertragungskondensators mit einem ersten Übertragungstaktsignal (784) verbunden ist,
einen ersten Transistor (932), der dafür ausgelegt ist, Ladung an den zweiten Anschluß des ersten Pumpenkondensators zu übertragen, und wobei der erste Transistor ein Gate, einen ersten Anschluß und einen zweiten Anschluß hat und das Gate des ersten Transistors mit dem zweiten Anschluß des ersten Übertragungskondensators verbunden ist und der zweite Anschluß des ersten Transistors mit dem zweiten Anschluß des ersten Pumpenkondensators verbunden ist,
einen zweiten Transistor (936), der dafür ausgelegt ist, wahlweise das Gate des ersten Transistors mit dem ersten Anschluß des ersten Transistors zu verbinden, und wobei der zweite Transistor ein Gate, einen ersten Anschluß und einen zweiten Anschluß hat, und das Gate des zweiten Transistors mit dem zweiten Anschluß des ersten Pumpenkondensators verbunden wird und der zweite Anschluß des zweiten Transistors mit dem Gate des ersten Transistors verbunden wird und der erste Anschluß des zweiten Transistors mit dem ersten Anschluß des ersten Transistors verbunden wird, und
wobei der Pumptaktschaltkreis aufweist:
eine Taktschaltung, die mit a) dem ersten Übertragungstaktsignal und b) dem ersten Anschluß des ersten Pumpenkondensators verbunden ist.

13. Ladungspumpe nach Anspruch 12, wobei der Taktschaltkreis einen Routing- bzw. Steuertransistor (810) mit einem Gate, einem ersten Anschluß und einem zweiten Anschluß aufweist, und wobei das Gate des Steuertransistors mit dem ersten Übertragungstaktsignal verbunden ist und der erste Anschluß des Steuertransistors mit dem ersten Anschluß des ersten Pumpenkondensators verbunden ist und der zweite Anschluß des Steuertransistors mit entweder einer Spannungsquelle, einer Stromquelle oder Masse verbunden ist.

14. Ladungspumpe nach Anspruch 12, welche weiterhin einen Verstärkungsschaltkreis aufweist, der mit zumindest einem der Folgenden, nämlich dem ersten Pumptaktsignal oder dem ersten Übertragungstaktsignal verbunden ist, wobei der Verstärkungsschaltkreis die Energie des zumindest einen, nämlich des ersten Pumptaktsignales oder des ersten Übertragungstaktsignales steigert.

15. Ladungspumpe nach Anspruch 12, welche weiterhin eine Diode aufweist, die einen ersten Anschluß und einen zweiten Anschluß hat, und wobei der erste Anschluß der Diode mit dem zweiten Anschluß des ersten Pumpenkondensators verbunden ist.

16. Ladungspumpe nach Anspruch 1, wobei eine erste aus der Mehrzahl von in Reihe angeordneten Ladungspumpenstufen (930) aufweist:
einen ersten Pumpenkondensator (942), der einen ersten Anschluß und einen zweiten Anschluß hat, und wobei der erste Anschluß des ersten Pumpenkondensators mit einem ersten Pumptaktsignal (724) verbunden ist,
einen ersten Übertragungskondensator (938), der einen ersten Anschluß und einen zweiten Anschluß hat, und der ersten Anschluß des ersten Übertragungskondensators mit einem ersten Übertragungstaktsignal (754) verbunden ist,
einen ersten Transistor (932), der dafür ausgelegt ist, Ladung zu dem zweiten Anschluß des ersten Pumpenkondensators zu übertragen, und wobei der erste Transistor ein Gate, einer ersten Anschluß und einen zweiten Anschluß hat, und wobei das Gate des ersten Transistors mit dem zweiten Anschluß des ersten Übertragungskondensators verbunden ist und der zweite Anschluß des ersten Transistors mit dem zweiten Anschluß des ersten Pumpenkondensators verbunden ist,
einen zweiten Transistor (936), der dafür ausgelegt ist, wahlweise das Gate des ersten Transistors mit dem ersten Anschluß des ersten Transistors zu verbinden, und wobei der zweite Transistor ein Gate, einen ersten Anschluß und einen zweiten Anschluß hat und das Gate des zweiten Transistors mit dem zweiten Anschluß des ersten Pumpenkondensators verbunden ist und der zweite Anschluß des zweiten Transistors mit dem Gate des ersten Transistors verbunden ist, und der erste Anschluß des zweiten Transistors mit dem ersten Anschluß des ersten Transistors verbunden ist, und
wobei eine zweite aus der Mehrzahl von in Reihe angeordneten Ladungspumpenstufen (950) aufweist:
einen zweiten Pumpenkondensator (962), der einen ersten Anschluß und einen zweiten Anschluß hat, und wobei der erste Anschluß des zweiten Pumpenkondensators mit einem zweiten Pumptaktsignal (764) verbunden ist,
einen zweiten Übertragungskondensator (758), der einen ersten Anschluß und einen zweiten Anschluß hat, und wobei der erste Anschluß des zweiten Übertragungskondensators mit einem zweiten Übertragungstaktsignal (744) verbunden ist,
einen dritten Transistor (952), der dafür ausgelegt ist, Ladung an den zweiten Anschluß des zweiten Pumpenkondensators zu übertragen, und wobei der dritte Transistor ein Gate, einen ersten Anschluß und einen zweiten Anschluß hat, und das Gate des dritten Transistors mit dem zweiten Anschluß des zweiten Übertragungskondensators verbunden ist und der erste Anschluß des dritten Kondensators mit dem zweiten Anschluß des ersten Transistors verbunden ist, und der zweite Anschluß des dritten Transistors mit dem zweiten Anschluß des zweiten Pumpkondensators verbunden ist,
einen vierten Transistor (956), der dafür ausgelegt ist, wahlweise das Gate des dritten Transistors mit dem ersten Anschluß des dritten Transistors zu verbinden und der vierte Transistor ein Gate, einen ersten Anschluß und einen zweiten Anschluß hat, und das Gate des vierten Transistors mit dem zweiten Anschluß des zweiten Pumpkondensators verbunden ist, und der zweite Anschluß des vierten Transistors mit dem Gate des dritten Transistors verbunden ist, und der erste Anschluß des vierten Transistors mit dem ersten Anschluß des dritten Transistors verbunden ist, und
wobei der Pumptaktschaltkreis eine Taktschaltung aufweist, die eines oder mehreres der Folgenden aufweist:
a) einen ersten Steuertransistor (810), der ein Gate, einen ersten Anschluß und einen zweiten Anschluß hat, und wobei das Gate des ersten Steuertransistors mit dem ersten Übertragungstaktsignal verbunden ist und der erste Anschluß des ersten Steuertransistors mit dem ersten Anschluß des ersten Pumpkondensators verbunden ist, und der zweite Anschluß des ersten Steuertransistors mit entweder einer Spannungsquelle, einer Stromquelle oder Masse verbunden ist,
b) einen zweiten Steuertransistor (820), der eine Gate, einen ersten Anschluß und einen zweiten Anschluß hat, und das Gate des zweiten Steuertransistors mit dem zweiten Übertragungstaktsignal verbunden ist und der erste Anschluß des zweiten Steuertransistors mit dem ersten Anschluß des ersten Pumpkondensators verbunden ist, und der zweite Anschluß des zweiten Steuertransistors mit entweder einer Spannungsquelle, einer Stromquelle oder Masse verbunden ist,
c) einen dritten Steuertransistor (830), der ein Gate, einen ersten Anschluß und einen zweiten Anschluß hat, und das Gate des dritten Steuertransistors mit dem ersten Übertragungstaktsignal verbunden ist und der erste Anschluß des dritten Steuertransistors mit dem ersten Anschluß des zweiten Pumpkondensators verbunden ist, und der zweite Anschluß des dritten Steuertransistors entweder mit einer Spannungsquelle, einer Stromquelle oder Masse verbunden ist, und
d) einen vierten Steuertransistor (840), der ein Gate, einen ersten Anschluß und einen zweiten Anschluß hat, und wobei das Gate des vierten Steuertransistors mit dem zweiten Übertragungstaktsignal verbunden ist, und der erste Anschluß des vierten Steuertransistors mit dem ersten Anschluß des zweiten Pumpkondensators verbunden ist, und der zweite Anschluß des vierten Steuertransistors entweder mit einer Spannungsquelle, einer Stromquelle oder Masse verbunden ist.

17. Ladungspumpe nach Anspruch 16, welche weiterhin einen Verstärkungsschaltkreis aufweist, der mit zumindest einem der Folgenden verbunden ist: dem ersten Pumptaktsignal, dem ersten Übertragungstaktsignal, dem zweiten Pumptaktsignal und dem zweiten Übertragungstaktsignal, wobei der Verstärkungsschaltkreis die Energie des zumindest einen, nämlich des ersten Pumptaktsignales, des ersten Übertragungstaktsignates, des zweiten Pumptaktsignales oder des zweiten Übertragungstaktsignales verstärkt.

18. Ladungspumpe nach Anspruch 16, welche weiterhin zumindest eines der Folgenden aufweist:
a) einen Verstärkungsschaltkreis (770), welcher das erste Übertragungstaktsignal mit dem Gate des dritten Steuertransistors verbindet, b) einen Verstärkungsschaltkreis (730), welcher das zweite Übertragungstaktsignal mit dem Gate des zweiten Steuertransistors verbindet, c) einen Verstärkungsschaltkreis (710), welcher das erste Übertragungstaktsignal mit dem Gate des ersten Steuertransistors verbindet, und d) einen Verstärkungsschaltkreis (750), welcher das zweite Übertragungstaktsignal mit dem Gate des vierten Steuertransistors verbindet.

19. Ladungspumpe nach Anspruch 17, wobei der Verstärkungsschaltkreis eines oder mehreres der Folgenden aufweist:
a) eine erste Mehrzahl von Invertierem (910), welche aufweisen:
einen ersten Invertierer (714), der einen Eingang und einen Ausgang hat, und wobei der Eingang des ersten Invertierers mit dem ersten Pumptaktsignal verbunden ist, einen zweiten Invertierer (716), der einen Eingang und einen Ausgang hat, und wobei der Eingang des zweiten Invertierers mit dem Ausgang des ersten Invertierers verbunden ist,
einen dritten Invertierer (718), der einen Eingang und einen Ausgang hat, und wobei der Eingang des dritten Invertierers mit dem Ausgang des zweiten Invertierers verbunden ist,
einen vierten Invertierer (720), der einen Eingang und einen Ausgang hat, und wobei der Eingang des vierten Invertierers mit dem Ausgang des dritten Invertierers verbunden ist, und wobei der Ausgang des vierten Invertierers mit dem ersten Anschluß des ersten Pumpkondensators verbunden ist,
b) eine zweite Mehrzahl von Invertierem (730), die aufweisen:
einen fünften Invertierer (734), der einen Eingang und einen Ausgang hat, und der Eingang des fünften Invertierers mit dem zweiten Übertragungstaktsignal verbunden ist,
einen sechsten Invertierer (736), der einen Eingang und einen Ausgang hat, und wobei der Eingang des sechsten Invertierers mit dem Ausgang des fünften Invertierers verbunden ist,
einen siebenten Invertierer (738), der einen Eingang und einen Ausgang hat, und wobei der Eingang des siebenten Invertierers mit dem Ausgang des sechsten Invertierers verbunden ist,
einen achten Invertierer (740), der einen Eingang und einen Ausgang hat, und wobei der Eingang des achten Invertierers mit dem Ausgang des siebenten Invertierers verbunden ist, und wobei der Ausgang des achten Invertierers mit dem ersten Anschluß des zweiten Übertragungskondensators verbunden ist,
c) eine dritte Mehrzahl von Invertiern (750), die aufweisen:
einen neunten Invertierer (754), der einen Eingang und einen Ausgang hat, und wobei der Eingang des neunten Invertierers mit dem zweiten Pumptaktsignal verbunden ist,
einen zehnten Invertierer (756), der einen Eingang und einen Ausgang hat, und wobei der Eingang des zehnten Invertierers mit dem Ausgang des neunten Invertierers verbunden ist,
einen elften Invertierer (758), der einen Eingang und einen Ausgang hat, und wobei der Eingang des elften Invertierers mit dem Ausgang des zehnten Invertierers verbunden ist,
einen zwölften Invertierer (760), der einen Eingang und einen Ausgang hat, und wobei der Eingang des zwöflten Invertierers mit dem Ausgang des elften Invertierers verbunden ist, und der Ausgang des zwölften Invertierers mit dem ersten Anschluß des zweiten Pumpkondensators verbunden ist, und
d) eine vierte Mehrzahl von Invertierem (770), welche aufweisen:
einen dreizehnten Invertierer (774), der einen Eingang und einen Ausgang hat, und wobei der Eingang des dreizehnten Invertierers mit dem ersten Übertragungstaktsignal verbunden ist,
einen vierzehnten Invertierer (776), der einen Eingang und einen Ausgang hat, und wobei der Eingang des vierzehnten Invertierers mit dem Ausgang des dreizehnten Invertierers verbunden ist,
einen fünfzehnten Invertierer (778), der einen Eingang und einen Ausgang hat, und wobei der Eingang des fünfzehnten Invertierers mit dem Ausgang des vierzehnten Invertierers verbunden ist,
einen sechzehnten Invertierer (780), der einen Eingang und einen Ausgang hat, und wobei der Eingang des sechzehnten Invertierers mit dem Ausgang des fünfzehnten Invertierers verbunden ist, und wobei der Ausgang des sechzehnten Invertierers mit dem ersten Anschluß des ersten Übertragungskondensators verbunden ist.

20. Ladungspumpe nach Anspruch 19, wobei das Gate des ersten Steuertransistors mit dem Ausgang des sechzehnten Invertierers verbunden ist, das Gate des zweiten Steuertransistors mit dem Ausgang des siebenten Invertierers verbunden ist, das Gate des dritten Steuertransistors mit dem Ausgang des fünfzehnten Invertierers verbunden ist und das Gate des vierten Steuertransistors mit dem Ausgang des achten Invertierers verbunden ist.

21. Ladungspumpe nach Anspruch 1, wobei eine erste aus der Mehrzahl von in Reihe angeordneten Ladungspumpenstufen (930) aufweist:
einen ersten Pumpkondensator (942), der einen ersten Anschluß und einen zweiten Anschluß hat, und wobei der erste Anschluß des ersten Pumpkondensators mit einem ersten Pumptaktsignal (724) verbunden ist,
einen ersten Übertragungskondensator (938), der einen ersten Anschluß und einen zweiten Anschluß hat, und wobei der erste Anschluß des ersten Übertragungskondensators mit einem ersten Übertragungstaktsignal (784) verbunden ist,
einen ersten Transistor (932), der dafür ausgelegt ist, Ladung zu dem zweiten Anschluß des ersten Pumpkondensators zu übertragen, und wobei der erste Transistor ein Gate, einen ersten Anschluß und einen zweiten Anschluß hat, und das Gate des ersten Transistors mit dem zweiten Anschluß des ersten Übertragungskondensators verbunden ist und der zweite Anschluß des ersten Transistors mit dem zweiten Anschluß des ersten Pumpkondensators verbunden ist,
einen zweiten Transistor (936), der dafür ausgelegt ist, wahlweise das Gate des ersten Transistors mit dem ersten Anschluß des ersten Transistors zu verbinden, und wobei der zweite Transistor ein Gate, einen ersten Anschluß und einen zweiten Anschluß hat, und das Gate des zweiten Transistors mit dem zweiten Anschluß des ersten Pumpkondensators verbunden ist, und der zweite Anschluß des zweiten Transistors mit dem Gate des ersten Transistors verbunden ist, und wobei der erste Anschluß des zweiten Transistors mit dem ersten Anschluß des ersten Transistors verbunden ist, und
wobei eine zweite der Mehrzahl von in Serie angeordneten Ladungspumpenstufen (950) aufweist:
einen zweiten Pumpkondensator (962), der einen ersten Anschluß und einen zweiten Anschluß hat, und wobei der erste Anschluß des zweiten Pumpkondensators mit einem zweiten Pumptaktsignal verbunden ist,
einen zweiten Übertragungskondensator (958), der einen ersten Anschluß und einen zweiten Anschluß hat, und wobei der erste Anschluß des zweiten Übertragungskondensators mit einem zweiten Übertragungstaktsignal verbunden ist,
einen dritten Transistor (952), der dafür ausgelegt ist, Ladung an den zweiten Anschluß des zweiten Pumpkondensators zu übertragen, und wobei der dritte Transistor ein Gate, einen ersten Anschluß und einen zweiten Anschluß hat, und das Gate des dritten Transistors mit dem zweiten Anschluß des zweiten Übertragungskondensators verbunden ist, und der erste Anschluß des dritten Transistors mit dem zweiten Anschluß des ersten Transistors verbunden ist, und der zweite Anschluß des dritten Transistors mit dem zweiten Anschluß des zweiten Pumpkondensators verbunden ist,
einen vierten Transistor (956), der dafür ausgelegt ist, wahlweise das Gate des dritten Transistors mit dem ersten Anschluß des dritten Transistors zu verbinden, und wobei der vierte Transistor ein Gate, einen ersten Anschluß und einen zweiten Anschluß hat, und das Gate des vierten Transistors mit dem zweiten Anschluß des zweiten Pumpkondensators verbunden ist, und der zweite Anschluß des vierten Transistors mit des Gate des dritten Transistors verbunden ist, und der erste Anschluß des vierten Transistors mit dem ersten Anschluß des dritten Transistors verbunden ist, und
wobei eine dritte aus der Mehrzahl von in Reihe angeordneten Ladungspumpenstufen (970) aufweist:
einen dritten Pumpkondensator (982), der einen ersten Anschluß und einen zweiten Anschluß hat, und wobei der erste Anschluß des dritten Pumpkondensators mit dem ersten Pumptaktsignal verbunden ist,
einen dritten Übertragungskondensator (978), der einen ersten Anschluß und einen zweiten Anschluß hat, und wobei der erste Anschluß des dritten Übertragungskondensators mit dem ersten Übertragungstaktsignal verbunden ist,
eine fünften Transistor (972), der dafür ausgelegt ist, Ladung auf den zweiten Anschluß des dritten Pumpkondensators zu ertragen, und wobei der fünfte Transistor ein Gate, einen ersten Anschluß und einen zweiten Anschluß hat, und das Gate des fünften Transistors mit dem zweiten Anschluß des dritten Übertragungskondensators verbunden ist und der erste Anschluß des fünften Transistors mit dem zweiten Anschluß des dritten Transistors verbunden ist, und der zweite Anschluß des fünften Transistors mit dem zweiten Anschluß des dritten Pumpkondensators verbunden ist,
einen sechsten Transistor (976), der dafür ausgelegt ist, wahlweise das Gate des fünften Transistors mit dem ersten Anschluß des fünften Transistors zu verbinden, und wobei der sechste Transistor ein Gate, einen ersten Anschluß und einen zweiten Anschluß hat, und das Gate des sechsten Transistors mit dem zweiten Anschluß des dritten Pumpkondensators verbunden ist, und der zweite Anschluß des sechsten Transistors mit dem Gate des fünften Transistors verbunden ist, und der erste Anschluß des sechsten Transistors mit dem ersten Anschluß des fünften Transistors verbunden ist, und
wobei der Zeittaktschaltkreis eine Taktschaltung aufweist, die eines oder mehreres der Folgenden aufweist:
a) einen ersten Steuertransistor (810), der ein Gate, einen ersten Anschluß und einen zweiten Anschluß hat, und wobei das Gate des ersten Steuertransistors mit dem ersten Übertragungstaktsignal verbunden ist und der erste Anschluß des ersten Steuertransistors mit zumindest einem der Folgenden, nämlich i) dem ersten Anschluß des ersten Pumpkondensators und ii) dem ersten Anschluß des dritten Pumpkondensators verbunden ist, und der zweite Anschluß des ersten Steuertransistors mit entweder einer Spannungsquelle, einer Stromquelle oder Masse verbunden ist,
b) einen zweiten Steuertransistor (820), der ein Gate, einen ersten Anschluß und einen zweiten Anschluß hat, und wobei das Gate des zweiten Steuertransistors mit dem zweiten Übertragungstaktsignal verbunden ist und der erste Anschluß des zweiten Steuertransistors mit zumindest einem der Folgenden verbunden ist, nämlich i) dem ersten Anschluß des ersten Pumpkondensators und ii) dem ersten Anschluß des dritten Pumpkondensators, und wobei der zweite Anschluß des zweiten Steuertransistors entweder mit einer Spannungsquelle, einer Stromquelle oder Masse verbunden ist,
c) einen dritten Steuertransistor (830), der ein Gate, einen ersten Anschluß und einen zweiten Anschluß hat, und wobei das Gate des dritten Steuertransistors mit dem ersten Übertragungstaktsignal verbunden ist und der erste Anschluß des dritten Steuertransistors mit dem ersten Anschluß des zweiten Pumpkondensators verbunden ist und der zweite Anschluß des dritten Steuertransistors mit entweder einer Spannungsquelle, einer Stromquelle oder Masse verbunden ist, und
d) einen vierten Steuertransistor (840), der ein Gate, einen ersten Anschluß und einen zweiten Anschluß hat, und wobei das Gate des vierten Steuertransistors mit dem zweiten Übertragungstaktsignal verbunden ist und der erste Anschluß des vierten Steuertransistors mit dem ersten Anschluß des zweiten Pumpkondensators verbunden ist, und der zweite Anschluß des vierten Steuertransistors entweder mit einer Spannungsquelle, einer Stromquelle oder Masse verbunden ist.

22. Mehrstufige Ladungspumpe nach Anspruch 21, welche weiterhin einen Verstärkungsschaltkreis aufweist, der zumindest mit einem der Folgenden, nämlich dem ersten Pumptaktsignal, dem ersten Übertragungstaktsignal, dem zweiten Pumptaktsignal und dem zweiten Übertragungstaktsignal verbunden ist, wobei der Verstärkungsschaltkreis die Energie des zumindest einen, nämlich des ersten Pumptaktsignales, des ersten Übertragungstaktsignales, des zweiten Pumptaktsignales und des zweiten Übertragungstaktsignales erhöht.

23. Mehrstufige Ladungspumpe nach Anspruch 21, welche weiterhin zumindest eines der Folgenden aufweist, nämlich a) einen Verstärkungsschaltkreis (770), weicher das erste Übertragungstaktsignal mit dem Gate des dritten Steuertransistors verbindet, b) einen Verstärkungsschaltkreis (730), welcher das zweite Übertragungstaktsignal mit dem Gate des zweiten Steuertransistors verbindet, c) einen Verstärkungsschaltkreis (710), welcher das erste Übertragungstaktsignal mit dem Gate des ersten Steuertransistors verbindet und d) einen Verstärkungsschaltkreis (750), welcher das zweite Übertragungstaktsignal mit dem Gate des vierten Steuertransistors verbindet.

24. Mehrstufige Ladungspumpe nach Anspruch 22, wobei der Verstärkungsschaltkreis eines oder mehreres der Folgenden aufweist:
a) eine erste Mehrzahl von Invertierem (910), welche aufweisen:
einen ersten Invertierer (714), der einen Eingang und einen Ausgang hat, und wobei der Eingang des ersten Invertierers mit dem ersten Pumptaktsignal verbunden ist, einen zweiten Invertierer (716), der einen Eingang und einen Ausgang hat, und wobei der Eingang des zweiten Invertierers mit dem Ausgang des ersten Invertierers verbunden ist,
einen dritten Invertierer (718), welcher einen Eingang und einen Ausgang hat, und wobei der Eingang des dritten Invertierers mit dem Ausgang des zweiten Invertierers verbunden ist,
einen vierten Invertierer (720), welcher einen Eingang und einen Ausgang hat, und wobei der Eingang des vierten Invertierers mit dem Ausgang des dritten Invertierers verbunden ist, und der Ausgang des vierten Invertierers mit zumindest einem der Folgenden, nämlich i) dem ersten Anschluß des ersten Pumpkondensators und ii) dem ersten Anschluß des dritten Pumpkondensators verbunden ist,
b) eine zweite Mehrzahl von Invertierem (730), die aufweisen:
einen fünften Invertierer (734), der einen Eingang und einen Ausgang hat, und wobei der Eingang des fünften Invertierers mit dem zweiten Übertragungstaktsignal verbunden ist,
einen sechsten Invertierer (736), der einen Eingang und einen Ausgang hat, und wobei der Eingang des sechsten Invertierers mit dem Ausgang des fünften Invertierers verbunden ist,
einen siebenten Invertierer (738), der einen Eingang und einen Ausgang hat, und wobei der Eingang des siebenten Invertierers mit dem Ausgang des sechsten Invertierers verbunden ist,
einen achten Invertierer (740), der einen Eingang und einen Ausgang hat, und wobei der Eingang des achten Invertierers mit dem Ausgang des siebenten Invertierers verbunden ist, und der Ausgang des achten Invertierers mit dem ersten Anschluß des zweiten Übertragungskondensators verbunden ist,
c) eine dritte Mehrzahl von Invertiern (750), welche aufweisen:
einen neunten Invertierer (754), der einen Eingang und einen Ausgang hat, und wobei der Eingang des neunten Invertierers mit dem zweiten Pumptaktsignal verbunden ist,
einen zehnten Invertierer (756), der einen Eingang und einen Ausgang hat, und wobei der Eingang des zehnten Invertierers mit dem Ausgang des neunten Invertierers verbunden ist,
einen elften Invertierer (758), der einen Eingang und einen Ausgang hat, und wobei der Eingang des elften Invertierers mit dem Ausgang des zehnten invertierers verbunden ist,
einen zwölften Invertierer (760), der einen Eingang und einen Ausgang hat, und wobei der Eingang des zwöflten Invertierers mit dem Ausgang des elften Invertierers verbunden ist, und der Ausgang des zwölften Invertierers mit dem ersten Anschluß des zweiten Pumpkondensators verbunden ist, und
d) eine vierte Mehrzahl von Invertierem (770), welche aufweisen:
einen dreizehnten Invertierer (774), der einen Eingang und einen Ausgang hat, und wobei der Eingang des dreizehnten Invertierers mit dem ersten Übertragungstaktsignal verbunden ist,
einen vierzehnten Invertierer (776), der einen Eingang und einen Ausgang hat, und wobei der Eingang des vierzehnten Invertierers mit dem Ausgang des dreizehnten Invertierers verbunden ist,
einen fünfzehnten Invertierer (778), der einen Eingang und einen Ausgang hat, und wobei der Eingang des fünfzehnten Invertierers mit dem Ausgang des vierzehnten Invertierers verbunden ist,
einen sechzehnten Invertierer (780), der einen Eingang und einen Ausgang hat, und wobei der Eingang des sechzehnten Invertierers mit dem Ausgang des fünfzehnten Invertierers verbunden ist, und der Ausgang des sechzehnten invertierers mit zumindest einem der Folgenden, nämlich i) dem ersten Anschluß des ersten Übertragungskondensators und ii) dem ersten Anschluß des dritten Übertragungskondensators verbunden ist.

25. Mehrstufige Ladungspumpe nach Anspruch 24, wobei das Gate des ersten Steuertransistors mit dem Ausgang des sechzehnten Invertierers verbunden ist, das Gate des zweiten Steuertransistors mit dem Ausgang des siebenten Invertierers verbunden ist, das Gate des dritten Steuertransistors mit dem Ausgang des fünfzehnten Invertierers verbunden ist und das Gate des vierten Steuertransistors mit dem Ausgang des achten Invertierers verbunden ist.

26. Integrierter Schaltkreis, welcher aufweist:
ein Halbleitersubstrat,
einen Pumpzeittaktschaltkreis (700) auf dem Substrat, der einen Zeittaktsignalausgang hat, und wobei der Zeittaktsignalausgang ein Zeittaktsignal liefert, und
eine Mehrzahl von in Reihe angeordneten Ladungspumpenstufen (930, 950, 970) auf dem Substrat, mit entsprechenden Übertragungsschaltkreisen und Pumpknoten (934, 954, 974), und wobei der Pumpknoten eines aus der Mehrzahl von in Reihe geschalteten Ladungspumpenstufen über einen Kondensator (742, 962, 982) mit dem Zeittaktsignal verbunden ist, wobei die Mehrzahl von in Reihe angeordneten Ladungspumpenstufen einen Ladungspumpenausgang (920) haben, wobei Ladung durch die Mehrzahl von Ladungspumpenstufen in Reaktion auf das Zeittaktsignal zu dem Ladungspumpenausgang gepumpt wird, und wobei das Zeittaktsignal durch eine erste Zeittaktsignalkomponente und eine zweite Zeittaktsignalkomponente getrieben bzw. angesteuert wird, und ein Einsetzen der ersten Zeittaktsignalkomponente um eine Verzögerung gegenüber einem Einsetzen der zweiten Zeittaktsignalkomponente getrennt ist.

27. Integrierter Schaltkreis nach Anspruch 26, wobei eine Richtung des Stromflusses des Zeittaktsignales sowohl während der ersten Zeittaktsignalkomponente als auch während der zweiten Zeittaktsignalkomponente konstant bleibt.

28. Integrierter Schaltkreis nach Anspruch 26, wobei eine Energie des Zeittaktsignales aufgrund des Einsetzens der zweiten Zeittaktsignalkomponente ansteigt.

29. Integrierter Schaltkreis nach Anspruch 28, wobei die Energie des Zeittaktsignales aufgrund des Einsetzens der zweiten Zeittaktsignalkomponente ansteigt, indem der Kondensator mit zumindest einem der Folgenden, nämlich einer Spannungsquelle, einer Stromquelle oder Masse verbunden wird.

30. Integrierter Schaltkreis nach Anspruch 26, welcher aufweist:
ein Speicherarray auf dem Substrat, und
eine Mehrzahl von Wortleitungen, die mit dem Speicherarray verbunden sind.

31. Verfahren zum Verringern der Größe eines Spitzenstromes, der in einer Ladungspumpe fließt, wobei die Ladungspumpe eine Mehrzahl von in Reihe angeordneten Ladungspumpenstufen (930, 950, 970) hat, welche entsprechende Übertragungsschaltkreise aufweisen, und mit Ladungspumpenknoten (934, 954, 974), wobei das Verfahren die Schritte aufweist:
(a) Treiben bzw. Ansteuern eines Ladungspumpenknotens in einer der Mehrzahl von Stufen in der Ladungspumpe mit einer ersten Zeittaktsignalkomponente, und
(b) Ansteuern des Ladungspumpenknotens mit einer zweiten Zeittaktsignalkomponente, wobei ein Einsetzen der ersten Zeittaktsignalkomponente und ein Einsetzen der zweiten Zeittaktsignalkomponente durch eine Verzögerung getrennt sind.

32. Verfahren nach Anspruch 31, wobei der betreffende Ladungspumpenknoten im Anschluß an das Einsetzen der zweiten Zeittaktsignalkomponente mit einer größeren Stromkapazität angesteuert wird.

33. Verfahren nach Anspruch 31, wobei
Schritt (a) das Ansteuern eines entsprechenden Ladungspumpenknotens in einer der Mehrzahl von Stufen aufweist, in dem eine erste Stromhandhabungseinrichtung aktiviert wird, welche mit dem betreffenden Ladungspumpenknoten über einen Kondensator verbunden ist, und nach einer Verzögerung, und
Schritt (b) das Ansteuern des betreffenden Ladungspumpenknotens durch Aktivieren einer zweiten Stromhandhabungseinrichtung aufweist, die mit dem betreffenden Ladungspumpenknoten durch den Kondensator verbunden ist, während die erste Stromhandhabungseinrichtung aktiviert bleibt.

34. Verfahren nach Anspruch 33, wobei die erste Stromhandhabungseinrichtung ein ersten Feldeffekttransistor ist und die zweite Stromhandhabungseinrichtung ein zweiter Feldeffekttransistor ist.

35. Verfahren nach Anspruch 31, wobei die erste Zeittaktsignalkomponente eine Polarität hat und die zweite Zeittaktsignalkomponente diese Polarität hat.

## Revendications

1. Pompe à charge, comprenant :
un circuit de cadencement de pompe (700) ayant une sortie de signal de cadencement et la sortie de signal de cadencement fournissant un signal de cadencement, et
une pluralité d'étages de pompes à charge agencés en série (930, 950, 970) comprenant des circuits de transfert et des noeuds de pompes (934, 954, 974) respectifs, le noeud de pompe d'un étage de ladite pluralité d'étages de pompes à charge étant relié à la sortie de signal de cadencement par l'intermédiaire d'un condensateur (942, 962, 982), ladite pluralité d'étages de pompes à charge agencés en série ayant une sortie de pompe à charge (920), où la charge est pompée vers la sortie de pompe à charge par l'intermédiaire de la pluralité d'étages de pompes à charge en réponse au signal de cadencement, et le signal de cadencement est entraîné par une première composante de signal de cadencement et par une seconde composante de signal de cadencement, et un début de ladite première composante de signal de cadencement est séparé par un délai par rapport au début de ladite seconde composante de signal de cadencement.

2. Pompe à charge selon la revendication 1, dans laquelle un sens de circulation de courant du signal de cadencement reste constant à la fois pendant la première composante de signal de cadencement et la seconde composante de signal de cadencement.

3. Pompe à charge selon la revendication 1, dans laquelle une puissance du signal de cadencement augmente en raison du début de la seconde composante de signal de cadencement.

4. Pompe à charge selon la revendication 3, dans laquelle la puissance du signal de cadencement augmente en raison du début de la seconde composante de signal de cadencement en reliant le condensateur à au moins l'une d'une source de tension, d'une source de courant et d'une masse.

5. Pompe à charge selon la revendication 1, dans laquelle la charge pompée vers la sortie de pompe à charge est positive.

6. Pompe à charge selon la revendication 1, dans laquelle
ledit circuit de cadencement de pompe comprend un circuit de cadencement de pompe ayant une pluralité d'entrées de signaux de cadencement (712, 732, 752, 772) et une pluralité de sorties de signaux de cadencement (722, 742, 762, 782), la pluralité d'entrées de signaux de cadencement recevant une pluralité de signaux de cadencement de pompes (620, 640, 660, 680) et une pluralité de sorties de signaux de cadencement fournissant une pluralité de signaux de cadencement de pompes amplifiés (724, 744, 764, 784) ; et
ladite pluralité d'étages de pompes à charge agencés. en série comprend une pluralité d'étages de pompes à charge agencés en série comprenant des circuits de transfert et des noeuds de pompes respectifs, les noeuds de pompes de ladite pluralité d'étages de pompes à charge étant reliés respectivement à la pluralité de sorties de signaux de cadencement par l'intermédiaire d'une pluralité de condensateurs, ladite pluralité d'étages de pompes à charge agencés en série ayant une sortie de pompe à charge, où la charge est pompée vers la sortie de pompe à charge par l'intermédiaire de la pluralité d'étages de pompes à charge en réponse à la pluralité de signaux de cadencement de pompes amplifiés, et au moins l'un de la pluralité de signaux de cadencement de pompes amplifiés est entraîné par une première composante de signal de cadencement et par une seconde composante de signal de cadencement, et un début de ladite première composante de signal de cadencement est séparé par un délai par rapport au début de ladite seconde composante de signal de cadencement.

7. Pompe à charge selon la revendication 6, dans laquelle le début de la première composante de signal de cadencement et le début de la seconde composante de signal de cadencement sont définis par des fronts de transition distincts définis par deux de la pluralité de signaux de cadencement de pompes.

8. Pompe à charge selon la revendication 6, dans laquelle le début de la première composante de signal de cadencement et le début de la seconde composante de signal de cadencement sont définis par une pluralité de signaux d'horloge.

9. Pompe à charge selon la revendication 6, dans laquelle un sens de circulation de courant d'au moins l'un de la pluralité de signaux de cadencement de pompes amplifiés reste constant à la fois pendant la première composante de signal de cadencement et la seconde composante de signal de cadencement.

10. Pompe à charge selon la revendication 6, dans laquelle une puissance d'au moins l'un de la pluralité de signaux de cadencement de pompes amplifiés augmente en raison du début de la seconde composante de signal de cadencement.

11. Pompe à charge selon la revendication 10, dans laquelle la puissance d'au moins l'un de la pluralité de signaux de cadencement de pompes amplifiés augmente en reliant l'un des condensateurs à au moins l'une d'une source de tension, d'une source de courant et d'une masse.

12. Pompe à charge selon la revendication 1, dans laquelle un premier de ladite pluralité d'étages de pompes à charge agencés en série (930) comprend :
un premier condensateur de pompe (942) ayant une première borne et une seconde borne, et la première borne du premier condensateur de pompe étant reliée à un premier signal d'horloge de pompe (724) ;
un premier condensateur de transfert (938) ayant une première borne et une seconde borne, la première borne du premier condensateur de transfert étant reliée à un premier signal d'horloge de transfert (784) ;
un premier transistor (932) conçu pour transférer une charge vers la seconde borne du premier condensateur de pompe, le premier transistor ayant une grille, une première borne et une seconde borne, la grille du premier transistor étant reliée à la seconde borne du premier condensateur de transfert et la seconde borne du premier transistor étant reliée à la seconde borne du premier condensateur de pompe;
un second transistor (936) conçu pour relier sélectivement la grille du premier transistor à la première borne du premier transistor, le second transistor ayant une grille, une première borne et une seconde borne, la grille du second transistor étant reliée à la seconde borne du premier condensateur de pompe, la seconde borne du second transistor étant reliée à la grille du premier transistor et la première borne du second transistor étant reliée à la première borne du premier transistor ; et
dans laquelle ledit circuit de cadencement de pompe comprend :
des circuits d'horloge reliés a) au premier signal d'horloge de transfert et b) à la première borne du premier condensateur de pompe.

13. Pompe à charge selon la revendication 12, dans laquelle les circuits d'horloge comprennent un transistor d'aiguillage (810) ayant une grille, une première borne et une seconde borne, la grille du transistor d'aiguillage étant reliée au premier signal d'horloge de transfert, la première borne du transistor d'aiguillage étant reliée à la première borne du premier condensateur de pompe et la seconde borne du transistor d'aiguillage étant reliée à l'une d'une source de tension, d'une source de courant et d'une masse.

14. Pompe à charge selon la revendication 12, comprenant en outre un circuit d'amplification relié à au moins l'un du premier signal d'horloge de pompe et du premier signal d'horloge de transfert, dans laquelle le circuit d'amplification augmente la puissance d'au moins l'un du premier signal d'horloge de pompe et du premier signal d'horloge de transfert.

15. Pompe à charge selon la revendication 12, comprenant en outre une diode ayant une première borne et une seconde borne, la première borne de la diode étant reliée à la seconde borne du premier condensateur de pompe.

16. Pompe à charge selon la revendication 1, dans laquelle un premier de ladite pluralité d'étages de pompes à charge agencés en série (930) comprend :
un premier condensateur de pompe (942) ayant une première borne et une seconde borne, la première borne du premier condensateur de pompe étant reliée à un premier signal d'horloge de pompe (724) ;
un premier condensateur de transfert (938) ayant une première borne et une seconde borne, la première borne du premier condensateur de transfert étant reliée à un premier signal d'horloge de transfert (754) ;
un premier transistor (932) conçu pour transférer une charge vers la seconde borne du premier condensateur de pompe, le premier transistor ayant une grille, une première borne et une seconde borne, la grille du premier transistor étant reliée à la seconde borne du premier condensateur de transfert et la seconde borne du premier transistor étant reliée à la seconde borne du premier condensateur de pompe;
un second transistor (936) conçu pour relier sélectivement la grille du premier transistor à la première borne du premier transistor, le second transistor ayant une grille, une première borne et une seconde borne, la grille du second transistor étant reliée à la seconde borne du premier condensateur de pompe, la seconde borne du second transistor étant reliée à la grille du premier transistor et la première borne du second transistor étant reliée à la première borne du premier transistor ; et
dans laquelle un second étage de ladite pluralité d'étages de pompes à charge agencés en série (950) comprend :
un second condensateur de pompe (962) ayant une première borne et une seconde borne, la première borne du second condensateur de pompe étant reliée à un second signal d'horloge de pompe (764) ;
un second condensateur de transfert (958) ayant une première borne et une seconde borne, la première borne du second condensateur de transfert étant reliée à un second signal d'horloge de transfert (744) ;
un troisième transistor (952) conçu pour transférer une charge vers la seconde borne du second condensateur de pompe, le troisième transistor ayant une grille, une première borne et une seconde borne, la grille du troisième transistor étant reliée à la seconde borne du second condensateur de transfert, la première borne du troisième transistor étant reliée à la seconde borne du premier transistor et la seconde borne du troisième transistor étant reliée à la seconde borne du second condensateur de pompe ;
un quatrième transistor (956) conçu pour relier sélectivement la grille du troisième transistor à la première borne du troisième transistor, le quatrième transistor ayant une grille, une première borne et une seconde borne, la grille du quatrième transistor étant reliée à la seconde borne du second condensateur de pompe, la seconde borne du quatrième transistor étant reliée à la grille du troisième transistor et la première borne du quatrième transistor étant reliée à la première borne du troisième transistor ; et
dans laquelle ledit circuit de cadencement de pompe comprend des circuits d'horloge comprenant un ou plusieurs éléments parmi :
a) un premier transistor d'aiguillage (810) ayant une grille, une première borne et une seconde borne, la grille du premier transistor d'aiguillage étant reliée au premier signal d'horloge de transfert, la première borne du premier transistor d'aiguillage étant reliée à la première borne du premier condensateur de pompe et la seconde borne du premier transistor d'aiguillage étant reliée à l'une d'une source de tension, d'une source de courant et d'une masse ;
b) un second transistor d'aiguillage (820) ayant une grille, une première borne et une seconde borne, la grille du second transistor d'aiguillage étant reliée au second signal d'horloge de transfert, la première borne du second transistor d'aiguillage étant reliée à la première borne du premier condensateur de pompe et la seconde borne du second transistor d'aiguillage étant reliée à l'une d'une source de tension, d'une source de courant et d'une masse ;
c) un troisième transistor d'aiguillage (830) ayant une grille, une première borne et une seconde borne, la grille du troisième transistor d'aiguillage étant reliée au premier signal d'horloge de transfert, la première borne du troisième transistor d'aiguillage étant reliée à la première borne du second condensateur de pompe et la seconde borne du troisième transistor d'aiguillage étant reliée à l'une d'une source de tension, d'une source de courant et d'une masse ; et
d) un quatrième transistor d'aiguillage (840) ayant une grille, une première borne et une seconde borne, la grille du quatrième transistor d'aiguillage étant reliée au second signal d'horloge de transfert, la première borne du quatrième transistor d'aiguillage étant reliée à la première borne du second condensateur de pompe et la seconde borne du quatrième transistor d'aiguillage étant reliée à l'une d'une source de tension, d'une source de courant et d'une masse.

17. Pompe à charge selon la revendication 16, comprenant en outre un circuit d'amplification relié à au moins l'un du premier signal d'horloge de pompe, du premier signal d'horloge de transfert, du second signal d'horloge de pompe et du second signal d'horloge de transfert, dans laquelle le circuit d'amplification augmente la puissance d'au moins l'un du premier signal d'horloge de pompe, du premier signal d'horloge de transfert, du second signal d'horloge de pompe et du second signal d'horloge de transfert.

18. Pompe à charge selon la revendication 16, comprenant en outre au moins un élément parmi : a) un circuit d'amplification (770) reliant le premier signal d'horloge de transfert à la grille du troisième transistor d'aiguillage, b) un circuit d'amplification (730) reliant le second signal d'horloge de transfert à la grille du second transistor d'aiguillage, c) un circuit d'amplification (710) reliant le premier signal d'horloge de transfert à la grille du premier transistor d'aiguillage, et d) un circuit d'amplification (750) reliant le second signal d'horloge de transfert à la grille du quatrième transistor d'aiguillage.

19. Pompe à charge selon la revendication 17, dans laquelle le circuit d'amplification comprend un ou plusieurs parmi :
a) une première pluralité d'inverseurs (910) comprenant :
un premier inverseur (714) ayant une entrée et une sortie, l'entrée du premier inverseur étant reliée au premier signal d'horloge de pompe ;
un second inverseur (716) ayant une entrée et une sortie, l'entrée du second inverseur étant reliée à la sortie du premier inverseur ;
un troisième inverseur (718) ayant une entrée et une sortie, l'entrée du troisième inverseur étant reliée à la sortie du second inverseur ;
un quatrième inverseur (720) ayant une entrée et une sortie, l'entrée du quatrième inverseur étant reliée à la sortie du troisième inverseur et la sortie du quatrième inverseur étant reliée à la première borne du premier condensateur de pompe ;
b) une seconde pluralité d'inverseurs (730) comprenant :
un cinquième inverseur (734) ayant une entrée et une sortie, l'entrée du cinquième inverseur étant reliée au second signal d'horloge de transfert ;
un sixième inverseur (736) ayant une entrée et une sortie, l'entrée du sixième inverseur étant reliée à la sortie du cinquième inverseur ;
un septième inverseur (738) ayant une entrée et une sortie, l'entrée du septième inverseur étant reliée à la sortie du sixième inverseur ;
un huitième inverseur (740) ayant une entrée et une sortie, l'entrée du huitième inverseur étant reliée à la sortie du septième inverseur et la sortie du huitième inverseur étant reliée à la première borne du second condensateur de transfert ;
c) une troisième pluralité d'inverseurs (750) comprenant :
un neuvième inverseur (754) ayant une entrée et une sortie, l'entrée du neuvième inverseur étant reliée au second signal d'horloge de pompe ;
un dixième inverseur (756) ayant une entrée et une sortie, l'entrée du dixième inverseur étant reliée à la sortie du neuvième inverseur ;
un onzième inverseur (758) ayant une entrée et une sortie, l'entrée du onzième inverseur étant reliée à la sortie du dixième inverseur ;
un douzième inverseur (760) ayant une entrée et une sortie, l'entrée du douzième inverseur étant reliée à la sortie du onzième inverseur et la sortie du douzième inverseur étant reliée à la première borne du second condensateur de pompe; et
d) une quatrième pluralité d'inverseurs (770) comprenant :
un treizième inverseur (774) ayant une entrée et une sortie, l'entrée du treizième inverseur étant reliée au premier signal d'horloge de transfert ;
un quatorzième inverseur (776) ayant une entrée et une sortie, l'entrée du quatorzième inverseur étant reliée à la sortie du treizième inverseur ;
un quinzième inverseur (778) ayant une entrée et une sortie, l'entrée du quinzième inverseur étant reliée à la sortie du quatorzième inverseur ;
un seizième inverseur (780) ayant une entrée et une sortie, la sortie du seizième inverseur étant reliée à la sortie du quinzième inverseur et la sortie du seizième inverseur étant reliée à la première borne du premier condensateur de transfert.

20. Pompe à charge selon la revendication 19, dans laquelle la grille du premier transistor d'aiguillage est reliée à la sortie du seizième inverseur, la grille du second transistor d'aiguillage est reliée à la sortie du septième inverseur, la grille du troisième transistor d'aiguillage est reliée à la sortie du quinzième inverseur et la grille du quatrième transistor d'aiguillage est reliée à la sortie du huitième inverseur.

21. Pompe à charge selon la revendication 1, dans laquelle un premier de ladite pluralité d'étages de pompes à charge agencés en série (930) comprend :
un premier condensateur de pompe (942) ayant une première borne et une seconde borne, la première borne du premier condensateur de pompe étant reliée à un premier signal d'horloge de pompe (724) ;
un premier condensateur de transfert (938) ayant une première borne et une seconde borne, la première borne du premier condensateur de transfert étant reliée à un premier signal d'horloge de transfert (784) ;
un premier transistor (932) conçu pour transférer une charge vers la seconde borne du premier condensateur de pompe, le premier transistor ayant une grille, une première borne et une seconde borne, la grille du premier transistor étant reliée à la seconde borne du premier condensateur de transfert et la seconde borne du premier transistor étant reliée à la seconde borne du premier condensateur de pompe;
un second transistor (936) conçu pour relier sélectivement la grille du premier transistor à la première borne du premier transistor, le second transistor ayant une grille, une première borne et une seconde borne, la grille du second transistor étant reliée à la seconde borne du premier condensateur de pompe, la seconde borne du second transistor étant reliée à la grille du premier transistor et la première borne du second transistor étant reliée à la première borne du premier transistor ; et
dans laquelle un second de ladite pluralité d'étages de pompes à charge agencés en série (950) comprend :
un second condensateur de pompe (962) ayant une première borne et une seconde borne, la première borne du second condensateur de pompe étant reliée à un second signal d'horloge de pompe ;
un second condensateur de transfert (958) ayant une première borne et une seconde borne, la première borne du second condensateur de transfert étant reliée à un second signal d'horloge de transfert ;
un troisième transistor (952) conçu pour transférer une charge vers la seconde borne du second condensateur de pompe et le troisième transistor ayant une grille, une première borne et une seconde borne, la grille du troisième transistor étant reliée à la seconde borne du second condensateur de transfert, la première borne du troisième transistor étant reliée à la seconde borne du premier transistor et la seconde borne du troisième transistor étant reliée à la seconde borne du second condensateur de pompe ;
un quatrième transistor (956) conçu pour relier sélectivement la grille du troisième transistor à la première borne du troisième transistor, le quatrième transistor ayant une grille, une première borne et une seconde borne, la grille du quatrième transistor étant reliée à la seconde borne du second condensateur de pompe, la seconde borne du quatrième transistor étant reliée à la grille du troisième transistor et la première borne du quatrième transistor étant reliée à la première borne du troisième transistor ; et
dans laquelle un troisième de ladite pluralité d'étages de pompes à charge agencés en série (970) comprend :
un troisième condensateur de pompe (982) ayant une première borne et une seconde borne, la première borne du troisième condensateur de pompe étant reliée au premier signal d'horloge de pompe ;
un troisième condensateur de transfert (978) ayant une première borne et une seconde borne, la première borne du troisième condensateur de transfert étant reliée au premier signal d'horloge de transfert ;
un cinquième transistor (972) conçu pour transférer une charge vers la seconde borne du troisième condensateur de pompe, le cinquième transistor ayant une grille, une première borne et une seconde borne, la grille du cinquième transistor étant reliée à la seconde borne du troisième condensateur de transfert, la première borne du cinquième transistor étant reliée à la seconde borne du troisième transistor et la seconde borne du cinquième transistor étant reliée à la seconde borne du troisième condensateur de pompe ;
un sixième transistor (976) conçu pour relier sélectivement la grille du cinquième transistor à la première borne du cinquième transistor, le sixième transistor ayant une grille, une première borne et une seconde borne, la grille du sixième transistor étant reliée à la seconde borne du troisième condensateur de pompe et la seconde borne du sixième transistor étant reliée à la grille du cinquième transistor et la première borne du sixième transistor étant reliée à la première borne du cinquième transistor ; et
dans laquelle ledit circuit de cadencement comprend des circuits d'horloge comprenant un ou plusieurs éléments parmi :
a) un premier transistor d'aiguillage (810) ayant une grille, une première borne et une seconde borne, la grille du premier transistor d'aiguillage étant reliée au premier signal d'horloge de transfert, la première borne du premier transistor d'aiguillage étant reliée à au moins l'une de i) la première borne du premier condensateur de pompe et ii) la première borne du troisième condensateur de pompe, la seconde borne du premier transistor d'aiguillage étant reliée à l'une d'une source de tension, d'une source de courant et d'une masse ;
b) un second transistor d'aiguillage (820) ayant une grille, une première borne et une seconde borne, la grille du second transistor d'aiguillage étant reliée au second signal d'horloge de transfert, la première borne du second transistor d'aiguillage étant reliée à au moins l'une de i) la première borne du premier condensateur de pompe et ii) la première borne du troisième condensateur de pompe, la seconde borne du second transistor d'aiguillage étant reliée à l'une d'une source de tension, d'une source de courant et d'une masse ;
c) un troisième transistor d'aiguillage (830) ayant une grille, une première borne et une seconde borne, la grille du troisième transistor d'aiguillage étant reliée au premier signal d'horloge de transfert, la première borne du troisième transistor d'aiguillage étant reliée à la première borne du second condensateur de pompe et la seconde borne du troisième transistor d'aiguillage étant reliée à l'une d'une source de tension, d'une source de courant et d'une masse; et
d) un quatrième transistor d'aiguillage (840) ayant une grille, une première borne et une seconde borne, la grille du quatrième transistor d'aiguillage étant reliée au second signal d'horloge de transfert, la première borne du quatrième transistor d'aiguillage étant reliée à la première borne du second condensateur de pompe et la seconde borne du quatrième transistor d'aiguillage étant reliée à l'une d'une source de tension, d'une source de courant et d'une masse.

22. Pompe à charge à étages multiples selon la revendication 21, comprenant en outre un circuit d'amplification relié à au moins l'un du premier signal d'horloge de pompe, du premier signal d'horloge de transfert, du second signal d'horloge de pompe et du second signal d'horloge de transfert, dans laquelle le circuit d'amplification augmente la puissance d'au moins l'un du premier signal d'horloge de pompe, du premier signal d'horloge de transfert, du second signal d'horloge de pompe et du second signal d'horloge de transfert.

23. Pompe à charge à étages multiples selon la revendication 21, comprenant en outre au moins l'un parmi : a) un circuit d'amplification (770) reliant le premier signal d'horloge de transfert à la grille du troisième transistor d'aiguillage, b) un circuit d'amplification (730) reliant le second signal d'horloge de transfert à la grille du second transistor d'aiguillage, c) un circuit d'amplification (710) reliant le premier signal d'horloge de transfert à la grille du premier transistor d'aiguillage, et d) un circuit d'amplification (750) reliant le second signal d'horloge de transfert à la grille du quatrième transistor d'aiguillage.

24. Pompe à charge à étages multiples selon la revendication 22, dans laquelle le circuit d'amplification comprend un ou plusieurs éléments parmi :
a) une première pluralité d'inverseurs (910) comprenant :
un premier inverseur (714) ayant une entrée et une sortie, l'entrée du premier inverseur étant reliée au premier signal d'horloge de pompe ;
un second inverseur (716) ayant une entrée et une sortie, l'entrée du second inverseur étant reliée à la sortie du premier inverseur ;
un troisième inverseur (718) ayant une entrée et une sortie, l'entrée du troisième inverseur étant reliée à la sortie du second inverseur ; un quatrième inverseur (720) ayant une entrée et une sortie, l'entrée du quatrième inverseur étant reliée à la sortie du troisième inverseur et la sortie du quatrième inverseur étant reliée à au moins l'une i) de la première borne du premier condensateur de pompe et ii) de la première borne du troisième condensateur de pompe ;
b) une seconde pluralité d'inverseurs (730) comprenant :
un cinquième inverseur (734) ayant une entrée et une sortie, l'entrée du cinquième inverseur étant reliée au second signal d'horloge de transfert ;
un sixième inverseur (736) ayant une entrée et une sortie, l'entrée du sixième inverseur étant reliée à la sortie du cinquième inverseur ;
un septième inverseur (738) ayant une entrée et une sortie, l'entrée du septième inverseur étant reliée à la sortie du sixième inverseur ;
un huitième inverseur (740) ayant une entrée et une sortie, l'entrée du huitième inverseur étant reliée à la sortie du septième inverseur et la sortie du huitième inverseur étant reliée à la première borne du second condensateur de transfert ;
c) une troisième pluralité d'inverseurs (750) comprenant :
un neuvième inverseur (754) ayant une entrée et une sortie, l'entrée du neuvième inverseur étant reliée au second signal d'horloge de pompe ;
un dixième inverseur (756) ayant une entrée et une sortie, l'entrée du dixième inverseur étant reliée à la sortie du neuvième inverseur ;
un onzième inverseur (758) ayant une entrée et une sortie, l'entrée du onzième inverseur étant reliée à la sortie du dixième inverseur ;
un douzième inverseur (760) ayant une entrée et une sortie, l'entrée du douzième inverseur étant reliée à la sortie du onzième inverseur et la sortie du douzième inverseur étant reliée à la première borne du second condensateur de pompe ; et
d) une quatrième pluralité d'inverseurs (770) comprenant :
un treizième inverseur (774) ayant une entrée et une sortie, l'entrée du treizième inverseur étant reliée à un premier signal d'horloge de transfert;
un quatorzième inverseur (776) ayant une entrée et une sortie, l'entrée du quatorzième inverseur étant reliée à la sortie du treizième inverseur ;
un quinzième inverseur (778) ayant une entrée et une sortie, l'entrée du quinzième inverseur étant reliée à la sortie du quatorzième inverseur ;
un seizième inverseur (780) ayant une entrée et une sortie, l'entrée du seizième inverseur étant reliée à la sortie du quinzième inverseur et la sortie du seizième inverseur étant reliée à au moins l'une i) de la première borne du premier condensateur de transfert et ii) de la première borne du troisième condensateur de transfert.

25. Pompe à charge à étages multiples selon la revendication 24, dans laquelle la grille du premier transistor d'aiguillage est reliée à la sortie du seizième inverseur, la grille du second transistor d'aiguillage est reliée à la sortie du septième inverseur, la grille du troisième transistor d'aiguillage est reliée à la sortie du quinzième inverseur et la grille du quatrième transistor d'aiguillage est reliée à la sortie du huitième inverseur.

26. Circuit intégré, comprenant :
un substrat de semiconducteur ;
un circuit de cadencement de pompe (700) sur le substrat comportant une sortie de signal de cadencement, la sortie de signal de cadencement fournissant un signal de cadencement ; et
une pluralité d'étages de pompes à charge agencés en série (930, 950, 970) sur le substrat, comprenant des circuits de transfert et des noeuds de pompes (934, 954, 974) respectifs, le noeud de pompe de l'un de la pluralité d'étages de pompes à charge reliés en série étant relié à la sortie de signal de cadencement par l'intermédiaire d'un condensateur (742, 962, 982), ladite pluralité d'étages de pompes à charge agencés en série ayant une sortie de pompe à charge (920), où une charge est pompée vers la sortie de pompe à charge par l'intermédiaire de la pluralité d'étages de pompes à charge en réponse au signal de cadencement et le signal de cadencement est attaqué par une première composante de signal de cadencement et par une seconde composante de signal de cadencement, et un début de ladite première composante de signal de cadencement est séparé par un délai par rapport à un début de ladite seconde composante de signal de cadencement.

27. Circuit intégré selon la revendication 26, dans lequel un sens de circulation de courant du signal de cadencement reste constant pendant à la fois la première composante de signal de cadencement et la seconde composante de signal de cadencement.

28. Circuit intégré selon la revendication 26, dans lequel une puissance du signal de cadencement augmente à cause du début de la seconde composante de signal de cadencement.

29. Circuit intégré selon la revendication 28, dans lequel la puissance du signal de cadencement augmente à cause du début de la seconde composante de signal de cadencement en reliant le condensateur à au moins l'une d'une source de tension, d'une source de courant et d'une masse.

30. Circuit intégré selon la revendication 26, comprenant :
une matrice de mémoire sur le substrat ; et
une pluralité de lignes de mots reliées à la matrice de mémoire.

31. Procédé de réduction de l'amplitude d'un courant de crête circulant dans une pompe à charge, la pompe à charge comportant une pluralité d'étages de pompes à charge agencés en série (930, 950, 970) comprenant des circuits de transfert et des noeuds de pompes à charge (934, 954, 974) respectifs, le procédé comprenant les étapes consistant à :
(a) attaquer un noeud de pompe à charge dans l'un de ladite pluralité d'étages dans la pompe à charge avec une première composante de signal de cadencement ; et
(b) attaquer le noeud de pompe à charge avec une seconde composante de signal de cadencement, dans lequel un début de la première composante de signal de cadencement et un début de la seconde composante de signal de cadencement sont séparés par un délai.

32. Procédé selon la revendication 31, dans lequel un noeud de pompe à charge particulier est attaqué avec une capacité de courant supérieure après le début de la seconde composante de signal de cadencement.

33. Procédé selon la revendication 31, dans lequel
l'étape (a) comprend l'attaque d'un noeud particulier de pompe à charge dans un étage de ladite pluralité d'étages en activant un premier dispositif de gestion de courant relié au noeud particulier de pompe à charge par l'intermédiaire d'un condensateur, et après un délai ; et
l'étape (b) comprend l'attaque du noeud particulier de pompe à charge en activant un second dispositif de gestion de courant relié au noeud particulier de pompe à charge par l'intermédiaire du condensateur alors que le premier dispositif de gestion de courant reste activé.

34. Procédé selon la revendication 33, dans lequel le premier dispositif de gestion de courant est un premier transistor à effet de champ et le second dispositif de gestion de courant est un second transistor à effet de champ.

35. Procédé selon la revendication 31, dans lequel la première composante de signal de cadencement a une polarité et la seconde composante de signal de cadencement a la polarité.
